(19) Europäisches Patentamt
European Patent Office
Office européen des brevets

(11) EP 0 691 342 B1

(12) FASCICULE DE BREVET EUROPEEN

(45) Date de publication et mention de la délivrance du brevet: **28.04.1999 Bulletin 1999/17**

(21) Numéro de dépôt: **95401607.7**

(22) Date de dépôt: **05.07.1995**

(51) Int. Cl.$^6$: **C07D 491/052**, A61K 31/445, A61K 31/40 // (C07D491/052, 311:00, 221:00), (C07D491/052, 311:00, 209:00)

(54) **Nouveaux dérivés de benzopyrane, leur procédé de préparation et les compositions pharmaceutiques qui les contiennent**

Benzopyranderivate, deren Herstellung und diese enthaltende pharmazeutische Zusammensetzungen

Benzopyran derivatives, process for their preparation and pharmaceutical compositions containing them

(84) Etats contractants désignés:
**AT BE CH DE DK ES FR GB GR IE IT LI LU NL PT SE**

(30) Priorité: **06.07.1994 FR 9408328**

(43) Date de publication de la demande:
**10.01.1996 Bulletin 1996/02**

(73) Titulaire: **ADIR ET COMPAGNIE**
**92415 Courbevoie Cédex (FR)**

(72) Inventeurs:
- **Lavielle, Gilbert**
  **F-78170 La Celle Saint Cloud (FR)**
- **Dubuffet, Thierry**
  **F-94550 Chevilly la Rue (FR)**
- **Millan, Mark**
  **F-75017 Paris (FR)**
- **Newman-Tancredi, Adrian**
  **F-78230 le Pecq (FR)**

(56) Documents cités:

| | |
|---|---|
| EP-A- 0 095 666 | EP-A- 0 410 535 |
| EP-A- 0 556 119 | WO-A-90/06927 |
| US-A- 3 888 946 | |

EP 0 691 342 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux dérivés de benzopyrane, leur procédé de préparation et les compositions pharmaceutiques qui les contiennent.

Les composés de la présente invention, outre le fait qu'ils soient nouveaux, présentent des propriétés particulièrement intéressantes en se fixant de manière sélective aux récepteurs sérotoninergiques 5-$HT_{2C}$ par rapport aux récepteurs 5-$HT_{2A}$. Cette propriété nouvelle n'a jamais été mise en évidence pour les composés les plus proches de l'Art Antérieur comme ceux par exemple décrits dans les brevets WO 9006927, EP 410535, EP 539209, US 3 888 946 ou bien encore EP 95666.

Il est bien établi que les voies ascendantes sérotoninergiques, dopaminergiques et adrénergiques projetant vers le système limbique et le cortex jouent un rôle déterminant dans le contrôle de l'humeur et dans l'étiologie et le traitement des maladies psychiatriques telles que la schizophrénie, la dépression, l'anxiété ainsi que l'agression et les autres troubles des impulsions (M.J. Millan et al., Drug News & Perspectives, 5, 397-406, 1992 ; A. Y. Deutch et al., Schizophrenia, 4, 121-156, 1991 ; H. Y. Meltzer and J.F. Nash, Pharmacol. Rev., 43, 587-604, 1991). Ces voies expriment leurs actions par une multitude de récepteurs différents et de plus en plus d'efforts sont faits pour identifier les types de récepteurs impliqués dans ces maladies. On espère donc parvenir, par une modulation de leur activité avec des agonistes ou des antagonistes, à corriger des désordres reflétant un dysfonctionnement des systèmes monoaminergiques.

En ce qui concerne la sérotonine (5-HT), au moins 7 types de récepteurs différents ont été clonés bien que, aujourd'hui, nos connaissances au niveau fonctionnel soient assez limitées pour plusieurs d'entre eux. Néanmoins, pour les deux sous-types de récepteurs 5-$HT_2$ qui sont présents au niveau cérébral, le 5-$HT_{2A}$ et le 5-$HT_{2C}$, il existe de bonnes indications pour qu'ils soient plus particulièrement impliqués dans le contrôle de l'humeur (J.F.W. Deakin, Pharmacol. Biochem. Behav., 29, 819-820, 1988) ainsi que dans la modulation de plusieurs fonctions physiologiques telles que l'appétit (G.A. Kennett et al., Eur. J. Pharmacol., 164, 445-454, 1989), le sommeil (C. Dugovic et al., Psychopharmacology, 97, 436-442, 1989), le comportement sexuel (H.H.G. Berendsen et al., Psychopharmacology, 101, 57-61, 1990), l'activité motrice (G.A. Kennett and G. Curzon, Psychopharmacology, 96, 93-100, 1988) et le fonctionnement cardiovasculaire (I.K. Anderson et al., Br. J. Pharmacol., 107, 1020-1028, 1992). En conséquence, chez l'animal, une activation des récepteurs 5-$HT_{2C}$ semble provoquer, par exemple, une diminution de l'activité motrice (I. Lucki et al., J. Pharmacol. Exp. Ther., 249, 155-164, 1989) et une réduction de la prise alimentaire (S.J. Kitchener and C.T. Dourish, Psychopharmacology, 113, 369-377, 1994), alors que chez l'animal ou chez l'homme, un antagonisme des récepteurs 5-$HT_{2A/2C}$ est associé à des effets anxiolytiques (G.A. Kennet et al., Eur. J. Pharmacol., 164, 445-454, 1989, D.L.S. Ceuleumans et al., Pharmacopsychiatry, 18, 303-305, 1985), antidépresseurs (F. Jenck et al., Eur. J. Pharmacol., 321, 223-229, 1993) et antischizophréniques (D.L.S. Ceuleumans et al., J. Pharmacol. Exp. Ther., 85, 329-332, 1985). Par ailleurs, le blocage des récepteurs 5-$HT_{2A/2C}$ semble être impliqué dans le profil atypique de l'antipsychotique, clozapine (A.Y. Deutch et al., Schizophrenia, 4, 121-156, 1991).

Vu leur très grande similarité, il a été extrêmement difficile de différencier les actions induites par les récepteurs 5-$HT_{2A}$ par rapport aux 5-$HT_{2C}$. De plus, pendant longtemps, il n'existait aucun antagoniste interactif de façon sélective avec des récepteurs 5-$HT_{2A}$ ou avec les récepteurs 5-$HT_{2C}$. Ainsi, la récente découverte d'un antagoniste sélectif 5-$HT_{2A}$, MDL 100,907, et d'un antagoniste sélectif 5-$HT_{2C}$, SB 200,646, a suscité beaucoup d'intérêt (Sorensen et al., J. Pharmacol. Exp. Ther., 1993, 266 (2), 684-691). Les premiers résultats obtenus avec le composé SB 200,646 montrant ses propriétés anxiolytiques laissent envisager un rôle particulièrement important des récepteurs 5-$HT_{2C}$ dans le contrôle de l'humeur (G.A. Kennett et al., Br. J. Pharmacol., 111, 797-802, 1994 ; G.A. Kennett et al., Eur. J. Pharmacol., 164, 445-454, 1989). Cette conviction est fortement renforcée par les résultats cliniques obtenus avec le mCPP qui se comporte comme un agoniste 5-$HT_{2C}$ et un antagoniste 5-$HT_{2A}$ (G.A. Kennett and G. Curzon , Br. J. Pharmacol., 94, 137-147, 1988 ; I. Lucki et al., J. Pharmacol. Exp. Ther., 249, 155-164, 1989 ; P.J. Conn and E. Sanders-Bush, J. Pharmacol. Exp. Ther., 242, 552-557, 1987) qui possède des propriétés anxiogéniques prononcées et qui exacerbe des états dépressifs, agressifs et psychotiques chez les malades (D.L. Murphy et al., Psychopharmacology, 98, 275-282, 1989 ; J.H. Krystal et al., Soc. Neurosci. Abst., 17, 354, 1991 ; J.P. Seibyl, Soc. Neurosci. Abst., 15, 1236, 1989).

Les composés décrits dans la présente invention se fixent de façon sélective aux récepteurs 5-$HT_{2C}$ par rapport aux 5-$HT_{2A}$ et sont ainsi susceptibles d'être utilisées dans le traitement des maladies telles que l'anxiété, la dépression, les troubles impulsifs (comme l'agression, B.A. McMillen, Drug. Develop. Persp., 12, 53-62, 1988), la schizophrénie, les troubles de l'appétit (comme l'anorexie), les maladies cardiovasculaires, le dysfonctionnement sexuel (H.H.G. Berendsen et al., Psychopharmacology, 101, 57-61, 1990), les attaques ischémiques cérébrales (F. Granier et al., Acta Psychiatr. Scand., 72, 67-74, 1985 ; W.D. Dietrich et al., J. Cereb. Blood Flow Metabol., 9, 812-820, 1989 ; J.A. Zivin, Neurology, 34, 469-474, 1984 ; J.A. Zivin, Neurology, 35, 584-587, 1985 ; K.M. Bode-Greuel et al., Stroke, 21, 164-166, 1990), l'abus de drogues (T.F. Meert and P.A.J. Janssen, Drug. Develop. Res., 25, 39-53, 1992 ; T.F. Meert and P.A.J. Janssen, Drug. Develop. Res., 25, 55-66, 1992 ; E.M. Sellers et al., Trends Pharmacol. Sci., 13, 69-75, 1992), les troubles du sommeil (C. Dugovic et al., Psychopharmacology, 97, 436-442, 1989) et la migraine (D.L. Murphy et al., Psychopharmacology, 98, 275-282, 1989).

Plus spécifiquement, la présente invention concerne les composés de formule (I) :

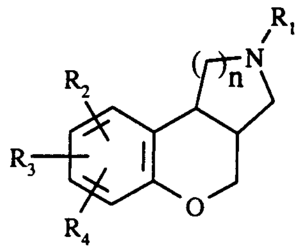

(I)

dans laquelle :

n représente 1 ou 2,

$R_1$ représente un atome d'hydrogène ou un groupement alkyle ($C_1$-$C_6$) linéaire ou ramifié, benzyle, acétyle, benzoyle, allyle, pyridinecarbonyle, pyridineméthyle, pyridineaminocarbonyle, phtalimidoalkyle ($C_1$-$C_6$) linéaire ou ramifié, (thiochroman-8-yloxy)alkyle ($C_1$-$C_4$) linéaire ou ramifié, (benzodioxanyloxy) alkyle ($C_1$-$C_4$) linéaire ou ramifié ou un groupement acylaminoalkyle ($C_1$-$C_6$) linéaire ou ramifié (dans lequel le groupement acyle est un groupement benzoyle, naphtylcarbonyle, thiénylcarbonyle, alkylcarbonyle ($C_1$-$C_6$) linéaire ou ramifié, furylcarbonyle, pyrrolylcarbonyle, pyridinylcarbonyle, cycloalkyle ($C_3$-$C_7$) carbonyle, chacun de ces groupements étant éventuellement substitué par un ou plusieurs atomes d'halogène ou groupements trihalogénométhyle, alkoxy ou hydroxy),

$R_2$, $R_3$ ou $R_4$, identiques ou différents représentent un atome d'hydrogène, d'halogène ou un groupement alkyle ($C_1$-$C_6$) linéaire ou ramifié, alkoxy ($C_1$-$C_6$) linéaire ou ramifié, hydroxy, acétyle, aminocarbonyle, aminométhyle, cyano, nitro, amino, phényle (substitué ou non par un ou plusieurs atomes d'halogène ou groupements hydroxy, alkoxy ($C_1$-$C_6$) linéaire ou ramifié, alkyle ($C_1$-$C_6$) linéaire ou ramifié ou trihalogénométhyle), furyle, pyridinyle, thiényle ou pyrrolyle,
ou bien,
$R_2$ et $R_3$, lorsqu'ils sont situés sur des carbones adjacents, forment avec les atomes de carbone qui les portent, un noyau furane ou phényle,

leurs isomères ainsi que leurs sels d'addition à un acide pharmaceutiquement acceptable.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique et camphorique.

L'invention concerne également le procédé de préparation des composés de formule (I). Ce procédé est caractérisé, lorsque les composés de formule (I) que l'on souhaite obtenir sont tels que n = 1, en ce que l'on utilise comme produit de départ une pyrrolidine de formule (II), sous forme de couple d'énantiomères, ou d'énantiomère pur :

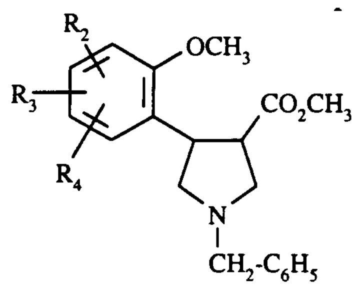

(II)

dans laquelle $R_2$, $R_3$ et $R_4$ sont tels que définis dans la formule (I),
composé de formule (II), qui, lorsqu'il est sous la forme d'un couple d'énantiomères, que l'on fait réagir avec de l'hydrure de lithium aluminium dans un solvant inerte pour conduire à la pyrrolidine de formule (III) :

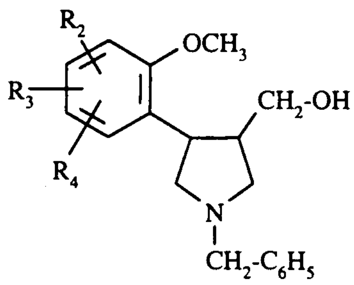

(III)

dans laquelle $R_2$, $R_3$ et $R_4$ ont la même signification que dans la formule (I), dont on transforme la fonction méthoxy en fonction hydroxy par réaction en présence d'éthanethiolate de sodium ou de tribromure de bore, puis que l'on fait réagir avec de l'acide chlorhydrique gazeux en présence de chlorure de thionyle, en milieu chloroformique, pour conduire au composé de formule (IV) :

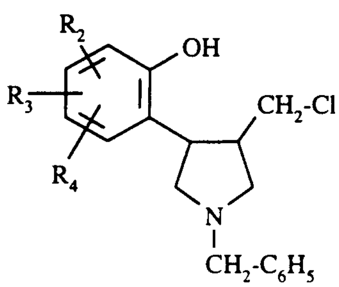

(IV)

dans laquelle $R_2$, $R_3$ et $R_4$ ont la même signification que dans la formule (I), qui est alors cyclisé en milieu basique, pour conduire au composé de formule (I/a), cas particulier des composés de formule (I) :

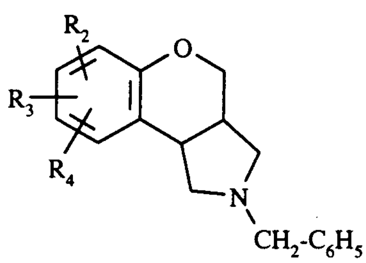

(I/a)

dans laquelle $R_2$, $R_3$ ou $R_4$ ont la même signification que dans la formule (I), composé de formule (I/a), dont on peut déprotéger, si on le souhaite, la fonction amine par hydrogénation catalytique, pour conduire au composé de formule (I/b), cas particulier des composés de formule (I) :

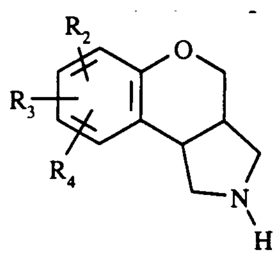

(I/b)

dans laquelle $R_2$, $R_3$ et $R_4$ ont la même signification que dans la formule (I), composé de formule (I/b), que l'on peut faire réagir, si on le souhaite, avec un dérivé halogéné :

R'$_1$X

dans laquelle :

X représente un atome d'halogène et

R'$_1$ représente un groupement alkyle ($C_1$-$C_6$) linéaire ou ramifié, acétyle, benzoyle, pyridinecarbonyle, pyridineméthyle, 3-pyridineaminocarbonyle, phtalimidoalkyle ($C_1$-$C_6$) linéaire ou ramifié,

pour conduire

- soit au composé de formule (I/c), cas particulier des composés de formule (I) :

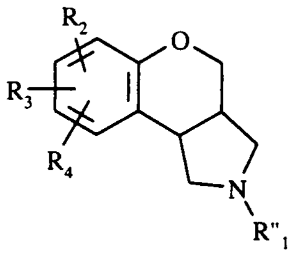

(I/c)

dans laquelle R$_2$, R$_3$ et R$_4$ ont la même signification que dans la formule (I) et R"$_1$ représente un groupement alkyle ($C_1$-$C_6$) linéaire ou ramifié, acétyle, benzoyle, pyridinecarbonyle, pyridineméthyle, 3-pyridineaminocarbonyle ou phtalimidoalkyle ($C_1$-$C_6$) linéaire ou ramifié,

- soit au composé de formule (V) :

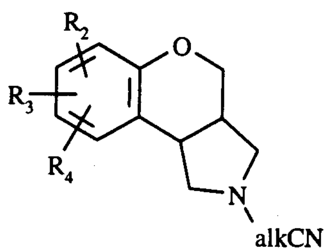

(V)

dans laquelle R$_2$, R$_3$ et R$_4$ ont la même signification que dans la formule (I) et alkCN représente un groupement cyanoalkyle ($C_1$-$C_6$) linéaire ou ramifié,

composé de formule (V) dont on réduit le groupement cyano en groupement amino et que l'on fait réagir sur un halogénure de benzoyle (substitué éventuellement par un atome d'halogène), pour conduire au composé de formule (I/d), cas particulier des composés de formule (I) :

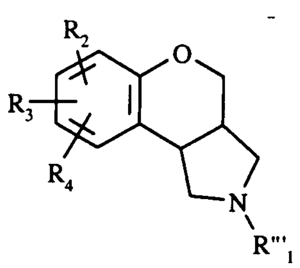

(I/d)

dans laquelle R$_2$, R$_3$ et R$_4$ ont la même signification que dans la formule (I) et R"'$_1$ représente un groupement benzoylaminoalkyle ($C_1$-$C_6$) linéaire ou ramifié (substitué éventuellement sur le noyau phényle par un atome d'halogène),

composé de formule (I/a), (I/b), (I/c) ou (I/d) :

- que l'on purifie éventuellement selon une technique classique de purification,

- dont on sépare, si on le souhaite, les énantiomères selon une technique classique de séparation,
- et que l'on transforme, le cas échéant, en ses sels d'addition à un acide pharmaceutiquement acceptable,
- étant entendu que les substituants $R_2$, $R_3$ et $R_4$ peuvent être introduits ou modifiés tout au long de la synthèse des composés de formule (I), selon des techniques classiques de la chimie organique.

Les composés de formule (II) utilisés comme produits de départ sont préparés selon le procédé décrit par K. ACHIWA et coll. (Chem. Pharm. Bull., 33(7), 2762-2766, 1985) en réalisant une cycloaddition d'un éthylénique de formule (IIa) :

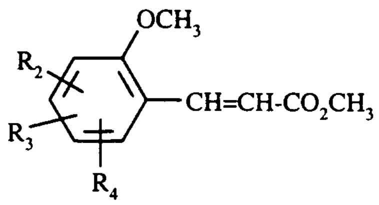

(IIa)

dans laquelle $R_2$, $R_3$ et $R_4$ ont la même signification que dans la formule (I), avec la N-benzyl-N-(méthoxyméthyl)triméthylsilylméthylamine en présence d'un catalyseur comme l'acide trifluoroacétique. Cette cycloaddition conduit selon la configuration de l'éthylénique de formule (IIa) utilisé, à une pyrrolidine de formule (II) dans laquelle les atomes d'hydrogène situés en position 3 et 4 sont en cis ou en trans l'un par rapport à l'autre.

Les composés de formules (IV) peuvent également être obtenus, lorsque la pyrrolidine possède des atomes d'hydrogène en position cis l'un par rapport à l'autre en réalisant une cycloaddition selon le procédé décrit par K. ACHIWA et coll. (cité plus haut) d'une coumarine de formule (VI) :

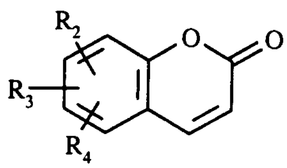

(VI)

dans laquelle $R_2$, $R_3$ et $R_4$ ont la même signification que dans la formule (I),
avec la N-benzyl-N-(méthoxyméthyl)triméthylsilylméthylamine en utilisant l'acide trifluoroacétique comme catalyseur, pour conduire au composé de formule (VII) :

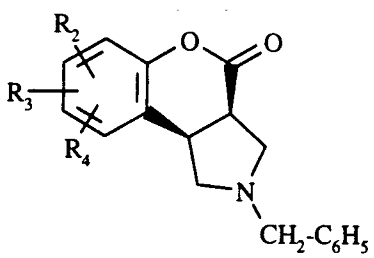

(VII)

dans laquelle $R_2$, $R_3$ et $R_4$ ont la même signification que dans la formule (I), qui subit alors une réduction en présence d'hydrure de lithium aluminium,
pour conduire au composé de formule (VIII) :

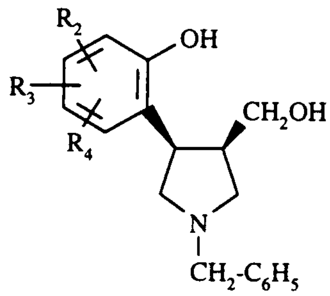

(VIII)

que l'on fait réagir avec de l'acide chlorhydrique gazeux en présence de chlorure de thionyle, pour conduire au composé de formule (IV).

Le procédé de préparation des composés de formule (I), pour lesquels n = 2, est caractérisé en ce que l'on utilise comme produit de départ le composé de formule (IX) :

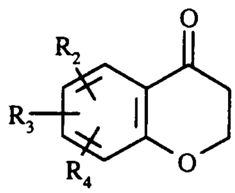

(IX)

dans laquelle $R_2$, $R_3$ et $R_4$ sont tels que définis dans la formule (I), que l'on fait réagir, selon la méthode décrite dans Can. J. Chem., 52, 2316, 1974, avec le bromure de méthylmagnésium puis avec l'acide p.toluènesulfonique,
pour conduire au composé de formule (X) :

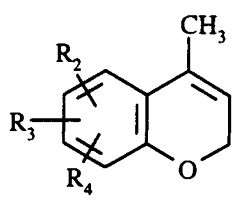

(X)

dans laquelle $R_2$, $R_3$ et $R_4$ ont la même signification que dans la formule (I), que l'on met en réaction avec la benzylamine en présence de formaldéhyde,
pour conduire au composé

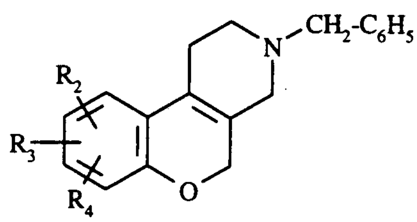

(XI)

de formule (XI) : dans laquelle $R_2$, $R_3$ et $R_4$ ont la même signification que dans la formule (I),
qui subit alors une hydrogénation catalytique, pour conduire au composé de formule (I/e), cas particulier des composés de formule (I) :

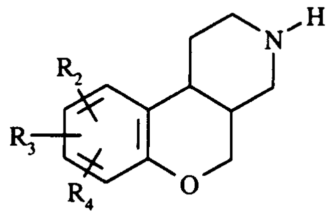

(I/e)

dans laquelle $R_2$, $R_3$ et $R_4$ ont la même signification que dans la formule (I), composé de formule (I/e), que l'on peut faire réagir, si on le souhaite, avec un dérivé halogéné de formule (XII) :

$$R'_{1a}\text{-}X \qquad (XII)$$

dans laquelle :

X représente un atome d'halogène et

$R'_{1a}$ représente un groupement alkyle ($C_1$-$C_6$) linéaire ou ramifié, acétyle, benzoyle, benzyle, pyridinecarbonyle, pyridineméthyle, 3-pyridineaminocarbonyle, phtalimidoalkyle ($C_1$-$C_6$) linéaire ou ramifié,

pour conduire

- soit au composé de formule (I/f), cas particulier des composés de formule (I) :

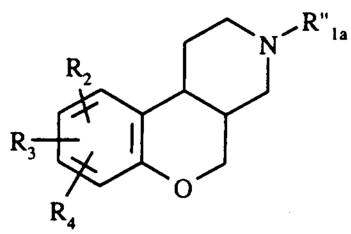

(I/f)

dans laquelle $R_2$, $R_3$ et $R_4$ ont la même signification que dans la formule (I) et $R''_{1a}$ représente un groupement alkyle ($C_1$-$C_6$) linéaire ou ramifié, acétyle, benzoyle, benzyle, pyridinecarbonyle, pyridineméthyle, 3-pyridineaminocarbonyle ou phtalimidoalkyle ($C_1$-$C_6$) linéaire ou ramifié,
- soit au composé de

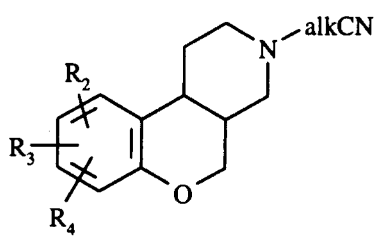

(XIII)

formule (XIII) : dans laquelle $R_2$, $R_3$ et $R_4$ ont la même signification que dans la formule (I) et alkCN représente un groupement cyanoalkyle ($C_1$-$C_6$) linéaire ou ramifié,
composé de formule (XIII) dont on réduit le groupement cyano en groupement amino et que l'on fait réagir sur un halogénure de benzoyle (substitué éventuellement par un atome d'halogène),
pour conduire au composé de formule (I/g), cas particulier des composés de formule (I) :

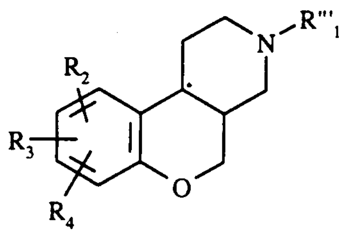

(I/g)

dans laquelle $R_2$, $R_3$ et $R_4$ ont la même signification que dans la formule (I) et $R'''_1$ représente un groupement benzoylaminoalkyle ($C_1$-$C_6$) linéaire ou ramifié (substitué éventuellement sur le noyau phényle par un atome d'halogène),
composé de formule (I/e), (I/f) ou (I/g) :

- que l'on purifie éventuellement selon une technique classique de purification,
- dont on sépare, si on le souhaite, les énantiomères selon une technique classique de séparation,
- et que l'on transforme, le cas échéant, en ses sels d'addition à un acide pharmaceutiquement acceptable,
- étant entendu que les substituants $R_2$, $R_3$ et $R_4$ peuvent être introduits ou modifiés tout au long de la synthèse des composés de formule (I), selon des techniques classiques de la chimie organique.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades et gels dermiques.

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 1 et 500 mg pour un traitement en 1 à 3 prises par 24 heures.

Les exemples suivants illustrent l'invention.
Les structures des composés décrits dans les exemples ont été confirmées par les techniques spectroscopiques usuelles.

La position des atomes d'hydrogène situés entre les deux hétérocycles est indiquée de la façon suivante :

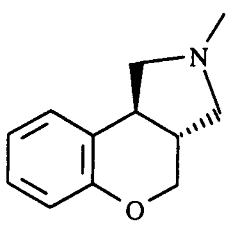

Trans-1,3,3a,4,9b-pentahydrobenzopyrano[3,4-c]pyrrole

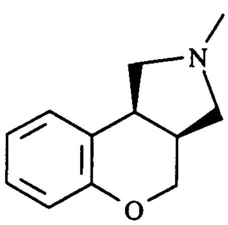

Cis-1,3,3a,4,9b-pentahydrobenzopyrano[3,4-c]pyrrole

Les préparations décrites ci-dessous conduisent à des produits de départ utilisés lors de la synthèse des composés de l'invention.

*Préparation A : [Trans-1-Benzyl-4-(2-méthoxyphényl)pyrrolidin-3-yl]carboxylate de méthyle*

Le produit attendu est obtenu selon le procédé décrit par K. ACHIWA et coll. (Chem. Pharm. Bull., 33(7), 2762-2766, 1985). A une solution contenant 120 mmoles de l'ester méthylique de l'acide trans-(2-méthoxy)cinnamique, et 0,1 ml d'acide trifluoroacétique dans 150 ml d'acétate d'éthyle refroidie à 5°C, sont ajoutées lentement 100 mmoles de N-benzyl-N-(méthoxyméthyl)triméthylsilylméthylamine. Le milieu réactionnel est porté de 30°C à 55°C pendant 75 minutes. 0,75 g de carbonate de potassium sont alors ajoutés et l'ensemble est maintenu sous agitation 15 minutes. Après filtration et évaporation des solvants, le résidu est repris par 100 ml d'acétate d'éthyle et la solution portée à 50°C. 110 mmoles d'acide oxalique en solution dans 100 ml d'acétone sont alors ajoutés sous vive agitation. Celle-ci est maintenue 15 heures. Le produit attendu sous forme d'oxalate est alors obtenu après filtration et rincé à l'éther. La base est obtenue après traitement de l'oxalate par deux équivalents de potasse 1N.
Infrarouge : $\nu_{CO}$ (nujol) = 1736 $cm^{-1}$

*Préparation B : [Trans-1-Benzyl-4-(2,6-diméthoxyphényl)pyrrolidin-3-yl] carboxylate de méthyle*

Le produit attendu est obtenu selon le procédé décrit dans la préparation A en utilisant comme produit de départ l'ester méthylique de l'acide trans-(2,6-diméthoxy)cinnamique.
Infrarouge : $\nu_{CO}$ (nujol) = 1736 $cm^{-1}$

*Préparation C : [Trans-1-Benzyl-4-(2,5-diméthoxyphényl)pyrrolidin-3-yl] carboxylate de méthyle*

Le produit attendu est obtenu selon le procédé décrit dans la préparation A en utilisant comme produit de départ l'ester méthylique de l'acide trans-(2,5-diméthoxy)cinnamique.
Infrarouge : $\nu_{CO}$ (nujol) = 1736 $cm^{-1}$

*Préparation D : Cis-2-Benzyl-1,3,3a,9b-tétrahydrobenzopyrano[3,4-c]pyrrole-4-one*

Le produit attendu est obtenu selon le procédé décrit dans la préparation A en utilisant comme produit de départ la coumarine.
Point de fusion (oxalate) : 170-175°C

*Préparation E : [Cis-1-Benzyl-4-(2,6-diméthoxyphényl)pyrrolidin-3-yl]carboxylate de méthyle*

Le produit attendu est obtenu selon le procédé décrit dans la préparation A en utilisant comme produit de départ l'ester méthylique de l'acide cis-(2,6-diméthoxy)cinnamique.
Infrarouge : $\nu_{CO}$ (nujol) = 1757 $cm^{-1}$

*Préparation F : [Trans-1-Benzyl-4-(2-méthoxy-4-chlorophényl)pyrrolidin-3-yl]carboxylate de méthyle*

Le produit attendu est obtenu selon le procédé décrit dans la préparation A en utilisant comme produit de départ l'ester méthylique de l'acide trans-(2-méthoxy-4-choro)cinnamique.
Infrarouge : $\nu_{CO}$ (nujol) = 1755 $cm^{-1}$

*Préparation G : Cis-2-benzyl-7-méthoxy-1,3,3a,9b-tétrahydrobenzopyrano[3,4-c]pyrrole-4-one*

Le produit attendu est obtenu selon le procédé décrit dans la préparation A en utilisant comme produit de départ la 7-méthoxy coumarine.
Point de fusion (oxalate) : 182-186°C

*Préparation H : [Trans-1-benzyl-4-(2,4-diméthoxyphényl)pyrrolidin-3-yl]carboxylate de méthyle*

Le produit attendu est obtenu selon le procédé décrit dans la préparation A en utilisant comme produit de départ l'ester méthylique de l'acide trans-(2,4-diméthoxy)cinnamique.

*Préparation I : [Trans-1-benzyl-4-(2,3-diméthoxyphényl)pyrrolidin-3-yl]carboxylate de méthyle*

Le produit attendu est obtenu selon le procédé décrit dans la préparation A en uilisant comme produit de départ l'ester méthylique de l'acide trans-(2,3-diméthoxy)cinnamique.

*Préparation J : Cis-2-benzyl-8-chloro-1,3,3a,9b-tétrahydrobenzopyrano[3,4-c]pyrrole-4-one*

Le produit attendu est obtenu selon le procédé décrit dans la préparation A en utilisant comme produit de départ la 6-chloro coumarine.
Point de fusion (oxalate) : 197°C

*Préparation K : [Trans-1-benzyl-4-(2-méthoxy-5-chlorophényl)pyrrolidin-3-yl]carboxylate de méthyle*

Le produit attendu est obtenu selon le procédé décrit dans la préparation A en utilisant comme produit de départ l'ester méthylique de l'acide trans(2-méthoxy-5-chloro)cinnamique.
Point de fusion (oxalate) : 144°C

*Préparation L : Cis-2-benzyl-6-chloro-1,3,3a,9b-tétrahydrobenzopyrano[3,4-c]pyrrole-4-one*

Le produit attendu est obtenu selon le procédé décrit dans la préparation A en utilisant comme produit de départ la 8-chloro coumarine.

*Préparation M : [Trans-1-benzyl-4-(2-méthoxy-3-chloro-phényl)pyrrolidin-3-yl]carboxylate de méthyle*

Le produit attendu est obtenu selon le procédé décrit dans la préparation A en utilisant comme produit de départ l'ester méthylique de l'acide trans(2-méthoxy-3-chloro)cinnamique.

*Préparation N : [Trans-1-benzyl-4-(2-méthoxy-5-bromo-phényl)pyrrolidin-3-yl]carboxylate de méthyle*

Le produit attendu est obtenu selon le procédé décrit dans la préparation A en utilisant comme produit de départ l'ester méthylique de l'acide trans(2-méthoxy-5-bromo)cinnamique.

*Préparation O : [Trans-1-benzyl-4-(2-méthoxy-napht-1-yl)pyrrolidin-3-yl]carboxylate de méthyle*

Le produit attendu est obtenu selon le procédé décrit dans la préparation A en utilisant comme produit de départ l'ester méthylique de l'acide trans 3-(2-méthoxy-napht-1-yl)acrylique.

*Préparation P : [Trans-1-benzyl-4-(1-méthoxy-napht-2-yl)pyrrolidin-3-yl]carboxylate de méthyle*

Le produit attendu est obtenu selon le procédé décrit dans la préparation A en utilisant comme produit de départ l'ester méthylique de l'acide trans 3-(1-méthoxy-napht-1-yl)acrylique.

*Préparation Q : Cis-16-benzyl-13,14,15,17-tétrahydro-11-oxa-12-one-16-aza-cyclopenta[a]phénantrène*

Le produit attendu est obtenu selon le procédé décrit dans la préparation A en utilisant comme produit de départ la benzo[h]chroman-2-one.

*Préparation R : Cis-2-benzyl-1,3,3a,11c-pentahydro-4-one-5-oxa-2-aza-cyclopenta[c]phénantrène*

Le produit attendu est obtenu selon le procédé décrit dans la préparation A en utilisant comme produit de départ la benzo[f]chromèn-3-one.

*Exemple 1 : Trans-2-Benzyl-1,3,3a,4,9b-pentahydro-(1)-benzopyrano[3,4-c]pyrrole, chlorhydrate*

*Stade A : Trans-1-Benzyl-3-hydroxyméthyl-4-(2-méthoxyphényl)pyrrolidine*

A 560 mmoles d'hydrure de lithiumaluminium dans 800 ml de tétrahydrofurane (THF), sous atmosphère d'azote, à 5°C, sont ajoutées 225 mmoles de [trans-1-benzyl-4-(2-méthoxyphényl)pyrrolidin-3-yl]carboxylate de méthyle (décrit dans la préparation A) en solution dans 500 ml de THF. Après 1 heure d'agitation à 5°C, 139 ml d'alcool isopropylique sont ajoutés lentement au mélange précédent puis 85,2 ml d'une solution saturée de chlorure de sodium. L'ensemble est agité lentement à température ambiante. Après filtration et évaporation des solvants, on obtient le produit attendu.

***Stade B : Trans-1-Benzyl-3-hydroxyméthyl-4-(2-hydroxyphényl)pyrrolidine***

A une solution préalablement préparée contenant 96 mmoles d'éthanethiolate de sodium dans 140 ml de diméthylformamide (DMF), sont ajoutées lentement 24 mmoles du composé obtenu au stade précédent en solution dans 120 ml de DMF. L'ensemble est porté 4 heures à 120°C.Après refroidissement, dilution à l'eau, extraction à l'éther, séchage et évaporation, on obtient le produit attendu.

***Stade C : Trans-1-Benzyl-3-chlorométhyl-4-(2-hydroxyphényl)pyrrolidine, chlorhydrate***

4,6 mmoles du composé obtenu au stade précédent sont dissoutes dans 100 ml de chloroforme. Après 10 minutes de barbotage d'acide chlorhydrique gaz, le milieu réactionnel est porté à reflux puis 13,8 mmoles de chlorure de thionyle sont additionnées. Le reflux est maintenu jusqu'à la fin du dégagement gazeux. Après évaporation du solvant, le résidu est repris par de l'éthanol puis évaporé. Le produit attendu précipite alors dans de l'éther.

***Stade D : Trans-2-Benzyl-1,3,3a,4,9b-pentahydro-(1)-benzopyrano[3,4-c]pyrrole, chlorhydrate***

2,95 mmoles du composé obtenu au stade précédent sont dissoutes dans 100 ml de THF et 10 ml d'hexaméthylphosphoretriamide (HMPT). 4 ml d'une solution 1,6M de butyllithium dans l'hexane sont alors ajoutés au mélange précédent. L'ensemble est maintenu 15 heures sous agitation. Après hydrolyse et évaporation des solvants, le résidu est repris par de l'eau et extrait à l'éther. Après séchage et évaporation des phases organiques, le produit attendu est obtenu par purification du résidu par chromatographie sur colonne de silice en utilisant comme éluant un mélange dichlorométhane/méthanol (97/3). La base est salifiée dans de l'éthanol chlorhydrique.

| *Microanalyse élémentaire :* | | | | |
|---|---|---|---|---|
| | *C %* | *H %* | *N %* | *Cl %* |
| *calculé* | *71,63* | *6,68* | *4,64* | *11,75* |
| *trouvé* | *71,31* | *6,61* | *4,70* | *12,04* |

***<u>Exemple 2</u> : Trans-2-Benzyl-9-hydroxy-1,3,3a,4,9b-pentahydro-(1)-benzopyrano [3,4-c]pyrrole, chlorhydrate***

***Stade A : Trans-1-Benzyl-3-hydroxyméthyl-4-(2,6-diméthoxyphényl)pyrrolidine***

Le produit attendu est obtenu selon le procédé décrit au stade A de l'exemple 1 en utilisant comme produit de départ le [trans-1-benzyl-4-(2,6-diméthoxyphényl)pyrrolidin-3-yl] carboxylate de méthyle décrit dans la préparation B.

***Stade B : Trans-1-Benzyl-3-chlorométhyl-4-(2,6-diméthoxyphényl)pyrrolidine***

Le produit attendu est obtenu selon le procédé décrit au stade C de l'exemple 1.

***Stade C : Trans-1-Benzyl-3-chlorométhyl-4-(2,6-dihydroxyphényl)pyrrolidine***

A 5,8 mmoles du composé obtenu au stade précédent en solution dans 100 ml de dichlorométhane, sont ajoutés 29 ml d'une solution 1M de tribromure de bore dans le dichlorométhane. Le mélange est porté à reflux pendant 5 heures. Après refroidissement, 77 ml d'éther éthylique puis 200 ml d'une solution saturée d'hydrogénocarbonate de sodium sont ajoutés. Après extraction à l'éther, les phases organiques sont lavées par une solution saturée d'hydrogénocarbonate de sodium, séchées et évaporées. Le produit attendu est obtenu après purification du résidu par chromatographie sur colonne de silice en utilisant comme éluant un mélange cyclohexane/acétate d'éthyle (80/20).

***Stade D : Trans-2-Benzyl-9-hydroxy-1,3,3a,4,9b-pentahydro-(1)-benzopyrano[3,4-c]pyrrole, chlorhydrate***

Le produit attendu est obtenu selon le procédé décrit au stade D de l'exemple 1.

| *Microanalyse élémentaire :* | | | | |
|---|---|---|---|---|
| | *C %* | *H %* | *N %* | *Cl %* |
| *calculé* | *68,09* | *6,03* | *4,41* | *11,17* |
| *trouvé* | *67,73* | *6,35* | *4,41* | *4,27* |

***Exemple 3** : Trans-2-Benzyl-9-méthoxy-1,3,3a,4,9b-pentahydro-(1)-benzopyrano[3,4-c]pyrrole, chlorhydrate*

***Stades A et B*** : Ces stades sont identiques aux stades A et B de l'exemple 2.

***Stade C : Trans-1-Benzyl-3-chlorométhyl-4-(2-hydroxy-6-méthoxyphényl)pyrrolidine***

A 28,9 mmoles du composé obtenu au stade précédent en solution dans 200 ml de dichlorométhane sont ajoutés 57,8 ml d'une solution 1M de tribromure de bore dans le dichlorométhane. Le milieu réactionnel est porté 45 minutes à reflux. Après refroidissement, addition de 800 ml d'eau, le pH est amené à 10. Après extraction au dichlorométhane, séchage et évaporation, le résidu est purifié par chromatographie sur colonne de silice en utilisant comme éluant le dichlorométhane. après relargage au méthanol puis à la soude 1N, on obtient le produit attendu.

***Stade D : Trans-2-Benzyl-9-méthoxy-1,3,3a,4,9b-pentahydro-(1)-benzopyrano[3,4-c]pyrrole, chlorhydrate***

A 9,3 mmoles d'hydrure de sodium dans 20 ml de THF, est ajoutée une solution contenant 7,8 mmoles du composé obtenu au stade précédent dans 20 ml de THF. Le milieu réactionnel est porté à reflux 3 heures puis hydrolysé. Après extraction à l'éther, séchage et évaporation, le produit attendu est obtenu par purification du résidu par chromatographie sur colonne de silice en utilisant comme éluant un mélange dichlorométhane/méthanol (95/5).

| *Microanalyse élémentaire :* | | | | |
|---|---|---|---|---|
| | *C %* | *H %* | *N %* | *Cl %* |
| *calculé* | *68,77* | *6,68* | *4,22* | *10,68* |
| *trouvé* | *68,34* | *6,47* | *4,51* | *11,21* |

***Exemple 4** : Trans-9-Méthoxy-1,3,3a,4,9b-pentahydro-(1)-benzopyrano[3,4-c]pyrrole, chlorhydrate*

2,2 mmoles du composé obtenu dans l'exemple 3 dans 100 ml d'éthanol et 30 ml d'eau sont hydrogénées pendant 1 heure à 45°C en présence de 70 mg de Palladium sur charbon comme catalyseur. Après évaporation des solvants, on obtient le produit attendu.

| *Microanalyse élémentaire :* | | | | |
|---|---|---|---|---|
| | *C %* | *H %* | *N %* | *Cl %* |
| *calculé* | *59,63* | *6,63* | *5,79* | *14,67* |
| *trouvé* | *58,93* | *6,09* | *5,85* | *15,14* |

***Exemple 5** : Trans-2-Acétyl-9-méthoxy-1,3,3a,4,9b-pentahydro-(1)-benzopyrano[3,4-c]pyrrole*

A 0,8 mmole du composé obtenu dans l'exemple 4 dans 30 ml de chloroforme, sont ajoutées successivement 1,66 mmoles de triéthylamine puis 0,8 mmole de chlorure d'acétyle. Après une heure d'agitation à température ambiante, le milieu réactionnel est hydrolysé. Après extraction au chloroforme, séchage et évaporation, le produit

attendu est obtenu après cristallisation du résidu dans de l'éther éthylique.

| *Microanalyse élémentaire :* | | | |
|---|---|---|---|
| | *C %* | *H %* | *N %* |
| *calculé* | *68,00* | *6,93* | *5,66* |
| *trouvé* | *67,21* | *6,57* | *5,83* |

***<u>Exemple 6</u> : Trans-2-Propyl-9-méthoxy-1,3,3a,4,9b-pentahydro-(1)-benzopyrano[3,4-c]pyrrole***

A 3,3 mmoles du composé obtenu dans l'exemple 4 en solution dans 20 ml d'acétonitrile et 10 ml d'acétone sont ajoutées successivement 6,6 mmoles de carbonate de potassium puis 3,3 mmoles de 1-bromopropane. Le milieu réactionnel est porté 15 heures à reflux. Après refroidissement, filtration, hydrolyse et extraction au dichlorométhane, les phases organiques sont séchées puis évaporées. Le produit attendu est obtenu après purification du résidu par chormatographie sur colonne de silice en utilisant comme éluant un mélange dichlorométhane/méthanol/ammoniaque (90/10/0,5). La base ainsi obtenue est salifiée dans de l'éthanol chlorhydrique.
<u>Point de fusion</u> : 233°C

| *Microanalyse élémentaire :* | | | | |
|---|---|---|---|---|
| | *C %* | *H %* | *N %* | *Cl %* |
| *calculé* | *63,48* | *7,81* | *4,94* | *12,45* |
| *trouvé* | *63,53* | *7,86* | *4,84* | *12,74* |

***<u>Exemple 7</u>*** : ***Trans-2-Allyl-9-méthoxy-1,3,3a,4,9b-pentahydro-(1)-benzopyrano[3,4-c]pyrrole***

A 3,3 mmoles du composé obtenu dans l'exemple 4 en solution dans 30 ml de chloroforme, sont ajoutées successivement 6,6 mmoles de triéthylamine puis 3,3 mmoles de bromure d'allyle. Le milieu réactionnel est porté une heure à reflux puis hydrolysé par de la soude 1N. Après extraction au chloroforme, les phases organiques sont réunies, séchées et évaporées. Le produit attendu est obtenu après purification du résidu par chromatographie sur colonne de silice en utilisant comme éluant un mélange dichlorométhane/méthanol/ammoniaque (95/5/0,5).

| *Microanalyse élémentaire :* | | | |
|---|---|---|---|
| | *C %* | *H %* | *N %* |
| *calculé* | *63,15* | *6,35* | *3,87* |
| *trouvé* | *61,59* | *6,41* | *3,88* |

***<u>Exemple 8</u>*** : ***Trans-2-[2-(4-Fluorobenzoylamino)éthyl]-9-méthoxy-1,3,3a,4,9b-pentahydro-(1)-benzopyrano[3,4c]pyrrole***

***Stade A : Trans-2-Cyanométhyl-9-méthoxy-1,3,3a,4,9b-pentahydro-(1)-benzopyrano[3,4-c]pyrrole***

A 4,14 mmoles du composé obtenu dans l'exemple 4 en solution dans 20 ml d'acétonitrile, sont ajoutées successivement 8,28 mmoles de carbonate de potassium puis une solution contenant 4,14 mmoles de bromoacétonitrile dans 20 ml d'acétonitrile. Le milieu réactionnel est porté 15 heures à reflux. Après refroidissement, filtration, le filtrat est hydrolysé et extrait au dichlorométhane. Les phases organiques sont séchées, évaporées et le produit attendu est obtenu après purification du résidu par chromatographie sur colonne de silice en utilisant comme éluant un mélange dichlorométhane/méthanol (98/2).

***Stade B : Trans-2-(2-Aminoéthyl)-9-méthoxy-1,3,3a,4,9b-pentahydro-(1)-benzopyrano[3,4-c]pyrrole***

1,5 mmoles du composé obtenu au stade précédent sont ajoutées à une solution contenant 3 mmoles d'hydrure de lithium aluminium dans 15 ml de THF à 5°C. Le milieu réactionnel est agité 90 minutes à cette température. Après addition de 0,17 ml d'eau, de 0,25 ml de soude 2N puis de 0,46 ml d'eau, le milieu est à nouveau agité pendant 3 heures puis filtré. Les solvants sont évaporés et le produit attendu est obtenu après purification du résidu par chromatographie sur colonne de silice en utilisant comme éluant un mélange dichlorométhane/méthanol/ammoniaque (80/20/2).

***Stade C : Trans-2-[2-(4-Fluorobenzoylamino)éthyl]-9-méthoxy-1,3,3a,4,9b-pentahydro-(1)-benzopyrano[3,4-c]pyrrole***

A 1 mmole du composé obtenu au stade précédent en solution dans 80 ml de chloroforme à 5°C, sont ajoutées 1 mmole de triéthylamine puis après 15 minutes d'agitation, 1 mmole de chlorure d'acide parafluorobenzoïque. Le milieu réactionnel est maintenu à 5°C pendant 90 minutes puis hydrolysé à l'aide de soude 1N. Après extraction au dichlorométhane, les phases organiques sont séchées, évaporées et le produit attendu est obtenu après purification du résidu par chromatographie sur colonne de silice.
La base est alors salifiée dans de l'éthanol chlorhydrique et le chlorhydrate cristallise dans le pentane.
Point de fusion : 202°C

| *Microanalyse élémentaire :* | | | | |
|---|---|---|---|---|
| | *C %* | *H %* | *N %* | *Cl %* |
| *calculé* | *61,99* | *5,95* | *6,88* | *8,71* |
| *trouvé* | *61,85* | *5,91* | *6,92* | *8,52* |

***Exemple 9*** : ***Trans-2-Benzyl-8-méthoxy-1,3,3a,4,9b-pentahydro-(1)-benzopyrano[3,4-c]pyrrole, chlorhydrate***

***Stade A : Trans-1-Benzyl-3-hydroxyméthyl-4-(2,5-diméthoxyphényl)pyrrolidine***

Le produit attendu est obtenu selon le procédé décrit au stade A de l'exemple 1 à partir du [trans-1-benzyl-4-(2,5-diméthoxyphényl)pyrrolidin-3-yl]carboxylate de méthyle décrit dans la préparation C.

***Stade B : Trans-1-Benzyl-3-hydroxyméthyl-4-(2-hydroxy-5-méthoxyphényl)pyrrolidine***

97 mmoles d'éthanethiol sont ajoutées à une solution contenant 97 mmoles d'hydrure de sodium dans 150 ml de DMF à 10°C. Après 15 minutes d'agitation à cette température, 24 mmoles du composé obtenu au stade A sont ajoutées et le milieu réactionnel est porté à 120°C pendant 3 heures. Après refroidissement, hydrolyse, extraction à l'éther puis au dichlorométhane, les phases organiques sont rassemblées, séchées et évaporées. Le produit attendu est obtenu après purification par chromatographie sur colonne de silice en utilisant comme éluant un mélange dichlorométhane/méthanol (95/5).

***Stade C : Trans-1-Benzyl-3-chlorométhyl-4-(2-hydroxy-5-méthoxyphényl)pyrrolidine***

Le produit attendu est obtenu selon le procédé décrit au stade C de l'exemple 1 à partir du composé obtenu au stade précédent.

***Stade D : Trans-2-Benzyl-8-méthoxy-1,3,3a,4,9b-pentahydro-(1)-benzopyrano[3,4-c]pyrrole, chlorhydrate***

Le produit attendu est obtenu selon le procédé décrit au stade D de l'exemple 1 à partir du composé obtenu au stade précédent.

| Microanalyse élémentaire : | | | | |
|---|---|---|---|---|
| | C % | H % | N % | Cl % |
| calculé | 68,77 | 6,68 | 4,22 | 10,68 |
| trouvé | 68,34 | 6,67 | 4,22 | 10,56 |

***Exemple 10* : Trans-8-Méthoxy-1,3,3a,4,9b-pentahydro-(1)-benzopyrano[3,4-c]pyrrole, chlorhydrate**

Le produit attendu est obtenu selon le procédé décrit dans l'exemple 4 à partir du composé obtenu dans l'exemple 9.

| Microanalyse élémentaire : | | | | |
|---|---|---|---|---|
| | C % | H % | N % | Cl % |
| calculé | 59,63 | 6,67 | 5,79 | 14,62 |
| trouvé | 59,58 | 6,60 | 5,77 | 14,30 |

***Exemple 11* : Trans-2-Propyl-8-méthoxy-1,3,3a,4,9b-pentahydro-(1)-benzopyrano[3,4-c]pyrrole, chlorhydrate**

Le produit attendu est obtenu selon le procédé décrit dans l'exemple 6 à partir du composé décrit dans l'exemple 10. La purification chromatographique est réalisée en utilisant comme éluant un mélange dichlorométhane/méthanol (95/5) et conduit au chlorhydrate.

| Microanalyse élémentaire : | | | | |
|---|---|---|---|---|
| | C % | H % | N % | Cl % |
| calculé | 63,48 | 7,88 | 4,94 | 12,49 |
| trouvé | 63,05 | 7,66 | 4,97 | 12,51 |

***Exemple 12* : Cis-2-Benzyl-1,3,3a,4,9b-pentahydro-(1)-benzopyrano[3,4-c]pyrrole, chlorhydrate**

***Stade A : Cis-1-Benzyl-3-hydroxyphényl-4-(2-hydroxyphényl)pyrrolidine***

125 mmoles de cis-2-benzyl-1,3,3a,9b-tétrahydro-benzopyrano[3,4-c]pyrrole-4-one (décrit dans la préparation D) sont ajoutées à une solution hétérogène contenant 310 mmoles d'hydrure de lithiumaluminium dans 800 ml de THF à 5°C. Le milieu réactionnel est maintenu sous agitation 3 heures à température ambiante avant d'être refroidi à +10°C. 120 ml d'alcool éthylique, 120 ml d'eau puis 40 ml d'une solution aqueuse d'hydroxyde de sodium à 40 % sont ajoutés successivement. Après filtration des sels, le filtrat est lavé par une solution saturée de bicarbonate de sodium. La phase organique est séchée et conduit après évaporation au produit attendu.

***Stade B : Cis-1-Benzyl-3-chlorométhyl-4-(2-hydroxyphényl)pyrrolidine, chlorhydrate***

Le produit attendu est obtenu selon le procédé décrit au stade C de l'exemple 1 à partir du composé décrit au stade précédent.

***Stade C : Cis-2-Benzyl-1,3,3a,4,9b-pentahydro-(1)-benzopyrano[3,4-c]pyrrole, chlorhydrate***

Le produit attendu est obtenu selon le procédé décrit au stade D de l'exemple 1 à partir du composé obtenu

au stade précédent.

| Microanalyse élémentaire : | | | | |
|---|---|---|---|---|
| | C % | H % | N % | Cl % |
| calculé | 71,63 | 6,68 | 4,64 | 11,75 |
| trouvé | 71,46 | 6,29 | 4,98 | 11,62 |

***Exemple 13** : Cis-1,3,3a,4,9b-Pentahydro-(1)-benzopyrano[3,4-c]pyrrole, chlorhydrate*

Le produit attendu est obtenu selon le procédé décrit dans l'exemple 4 à partir du composé obtenu dans l'exemple 12.

| Microanalyse élémentaire : | | | | |
|---|---|---|---|---|
| | C % | H % | N % | Cl % |
| calculé | 62,41 | 6,67 | 6,62 | 16,75 |
| trouvé | 61,77 | 6,47 | 6,43 | 16,54 |

***Exemple 14** : Cis-2-Benzyl-9-hydroxy-1,3,3a,4,9b-pentahydro-(1)-benzopyrano[3,4-c]pyrrole, chlorhydrate*

***Stade A : Cis-1-Benzyl-3-hydroxyméthyl-4-(2,6-diméthoxyphényl)pyrrolidine***

Le produit attendu est obtenu selon le procédé décrit au stade A de l'exemple 12 à partir du [cis-1-benzyl-4-(2,6-diméthoxyphényl)pyrrolidin-3-yl]carboxylate de méthyle décrit dans la préparation E.

***Stade B : Cis-1-Benzyl-3-chlorométhyl-4-(2,6-diméthoxyphényl)pyrrolidine***

Le produit attendu est obtenu selon le procédé décrit au stade C de l'exemple 1 à partir du composé décrit au stade précédent.

***Stade C : Cis-1-Benzyl-3-chlorométhyl-4-(2,6-dihydrophényl)pyrrolidine, chlorhydrate***

A 8,9 mmoles du composé obtenu au stade précédent dans 170 ml de dichlorométhane, sont ajoutés 44,5 ml d'une solution 1M de tribromure de bore dans le dichlorométhane. Le milieu réactionnel est porté 8 heures à reflux puis traité par de la soude concentrée pendant une heure. Le milieu est alors neutralisé à l'aide d'acide chlorhydrique. Après extraction au dichlorométhane, le produit attendu est obtenu après purification du résidu par chromatographie sur colonne de silice en utilisant comme éluant un mélange dichlorométhane/méthanol (95/5).

***Stade D : Cis-2-Benzyl-9-hydroxy-1,3,3a,4,9b-pentahydro-(1)-benzopyrano[3,4-c]pyrrole, chlorhydrate***

Le produit attendu est obtenu selon le procédé décrit au stade D de l'exemple 1.

| Microanalyse élémentaire : | | | | |
|---|---|---|---|---|
| | C % | H % | N % | Cl % |
| calculé | 68,03 | 6,34 | 4,41 | 4,26 |
| trouvé | 67,43 | 6,50 | 4,38 | 10,66 |

***Exemple 15** : Cis-2-Benzyl-9-méthoxy-1,3,3a,4,9b-pentahydro-(1)-benzopyrano[3,4-c]pyrrole, chlorhydrate*

11,6 mmoles du composé obtenu dans l'exemple 14 dans 50 ml de DMF sont ajoutées à une solution contenant 14 mmoles d'hydrure de sodium dans 50 ml de DMF. Après 30 minutes d'agitation, on ajoute 11,6 mmoles d'iodure de méthyle. Après 1 heure à température ambiante puis hydrolyse, les solvants sont évaporés. Le résidu est alors repris à l'eau. Après extraction à l'éther, séchage et évaporation, le produit attendu est obtenu après purification du résidu par chromatographie sur colonne de silice en utilisant comme éluant un mélange cyclohexane/acétate d'éthyle (75/25).

| *Microanalyse élémentaire :* | | | | |
|---|---|---|---|---|
| | *C %* | *H %* | *N %* | *Cl %* |
| *calculé* | *68,77* | *6,68* | *4,22* | *10,68* |
| *trouvé* | *68,66* | *4,48* | *4,45* | *10,97* |

***Exemple 16** : Cis-9-Méthoxy-1,3,3a,4,9b-pentahydro-(1)-benzopyrano[3,4-c]pyrrole, chlorhydrate*

Le produit attendu est obtenu selon le procédé décrit dans l'exemple 4 à partir du composé décrit dans l'exemple 15.

| *Microanalyse élémentaire :* | | | | |
|---|---|---|---|---|
| | *C %* | *H %* | *N %* | *Cl %* |
| *calculé* | *59,63* | *6,67* | *5,79* | *14,67* |
| *trouvé* | *59,97* | *6,69* | *5,93* | *13,87* |

***Exemple 17** : Cis-2-Acétyl-9-méthoxy-1,3,3a,4,9b-pentahydro-(1)-benzopyrano[3,4-c]pyrrole*

Le produit attendu est obtenu selon le procédé décrit dans l'exemple 5 à partir du composé décrit dans l'exemple 16.

| *Microanalyse élémentaire :* | | | |
|---|---|---|---|
| | *C %* | *H %* | *N %* |
| *calculé* | *68,00* | *6,93* | *5,68* |
| *trouvé* | *67,89* | *6,94* | *5,49* |

***Exemple 18** : Trans-2-Benzyl-7-chloro-1,3,3a,4,9b-pentahydro-(1)-benzopyrano[3,4-c]pyrrole, chlorhydrate*

***Stade A : Trans-1-Benzyl-3-hydroxyméthyl-4-(2-méthoxy-4-chlorophényl)pyrrolidine***

Le produit attendu est obtenu selon le procédé décrit au stade A de l'exemple 1 en utilisant comme produit de départ le [trans-1-benzyl-4-(2-méthoxy-4-chlorophényl)pyrrolidin-3-yl]carboxylate de méthyle décrit dans la préparation F.

***Stade B : Trans-1-Benzyl-3-hydroxyméthyl-4-(2-hydroxy-4-chlorophényl)pyrrolidine***

Le produit attendu est obtenu selon le procédé décrit au stade B de l'exemple 9 à partir du composé obtenu au stade précédent.

*Stade C : Trans-1-Benzyl-3-chlorométhyl-4-(2-hydroxy-4-chlorophényl)pyrrolidine*

Le produit attendu est obtenu selon le procédé décrit au stade C de l'exemple 1 à partir du composé obtenu au stade précédent.

*Stade D : Trans-2-Benzyl-7-chloro-1,3,3a,4,9b-pentahydro-(1)-benzopyrano[3,4-c]pyrrole, chlorhydrate*

Le produit attendu est obtenu selon le procédé décrit au stade D de l'exemple 3 (en utilisant deux équivalents d'hydrure de sodium) à partir du composé obtenu au stade précédent.

| *Microanalyse élémentaire :* | | | | |
|---|---|---|---|---|
| | *C %* | *H %* | *N %* | *Cl %* |
| *calculé* | *64,29* | *5,70* | *4,17* | *21,09* |
| *trouvé* | *64,25* | *5,72* | *4,03* | *21,27* |

***Exemple 19*** : ***Cis-2-[2-(thiochroman-8-yloxy)éthyl]-1,3,3a,9b-pentahydro-(1)-benzopyrano[3,4-c]pyrrole, chlorhydrate***

A 4,7 mmoles de chlorhydrate de cis-1,3,3a,4,9b-pentahydro-(1)-benzopyrano[3,4-c]pyrrole décrit dans l'exemple 13 dans 50 ml d'acétonitrile, on ajoute 9,4 mmoles de carbonate de potassium. Après une agitation de 15 min, on ajoute successivement 0,5 mmoles d'iodure de potassium puis 4,7 mmoles de 1-chloro-2-(thiochroman-8-yloxy)éthane en solution dans 50 ml d'acétonitrile. Le milieu réactionnel est porté au reflux pendant 24 heures puis évaporé, repris dans l'eau, extrait au dichlorométhane et séché sur sulfate de magnésium avant d'être filtré. Les solvants sont évaporés. Le produit brut est purifié par chromatographie sur colonne de silice (éluant: $CH_2Cl_2$/MeOH : 97/3). Le produit est ensuite salifié par une solution d'HCl dans l'éthanol.

| *Microanalyse élémentaire :* | | | | | |
|---|---|---|---|---|---|
| | *C %* | *H %* | *N %* | *Cl %* | *S %* |
| *calculé* | *65,41* | *6,49* | *3,47* | *8,78* | *7,94* |
| *trouvé* | *65,08* | *6,31* | *3,58* | *8,23* | *8,23* |

***Exemple 20* : *Cis-2-[(pyrid-3-yl)aminocarbonyl]-1,3,3a,4,9b-pentahydro-(1)-benzopyrano[3,4-c]pyrrole, chlorhydrate***

A 30 mmoles du produit obtenu à l'exemple 16 dans 50 ml de dichlorométhane, on ajoute 0,15 mmoles d'isocyanate de pyridyn-3-yl. Après une agitation de 48 heures, le produit est filtré puis chromatographié sur colonne de silice (éluant : dichlorométhane/méthanol : 95/5). Le produit obtenu est salifié à l'aide d'une solution d'acide chlorhydrique éthanolique.

| *Microanalyse élémentaire :* | | | | |
|---|---|---|---|---|
| | *C %* | *H %* | *N %* | *Cl %* |
| *calculé* | *59,75* | *5,57* | *11,61* | *9,80* |
| *trouvé* | *60,06* | *5,56* | *11,38* | *8,94* |

***Exemple 21** : Cis-2-benzyl-7-méthoxy-1,3,3a,4,9b-pentahydro-(1)-benzopyrano[3,4-c]pyrrole, chlorhydrate*

***Stade A : Cis-1-benzyl-3-hydroxyméthyl-4-(2-hydroxy-4-méthoxyphényl)pyrrolidine***

A 230 mmoles d'hydrure de lithiumaluminium dans 800 ml de THF, sous atmosphère d'azote, sont ajoutées 180 mmoles de cis-2-benzyl-7-méthoxy-1,3,3a,9b-tétrahydrobenzopyrano [3,4-c]pyrrole-4-one décrit dans la préparation G à + 5°C. Le milieu réactionnel est maintenu à + 10°C pendant une heure avant d'être hydrolysé, filtré sur célite. La phase organique est séchée et conduit après évaporation au produit attendu.

***Stade B : Cis-2-benzyl-7-méthoxy-1,3,3a,4,9b-pentahydro-(1)-benzopyrano[3,4-c]pyrrole, chlorhydrate***

A 93 mmoles du composé obtenu au stade A dans 700 ml de tétrahydrofurane (THF), on ajoute, successivement, 93 mmoles d'azodicarboxylate de diéthyle et 93 mmoles de triphénylphosphine. Le milieu réactionnel est agité pendant 3 heures à température ambiante puis les solvants sont évaporés. Le produit brut est purifié par chromatographie sur colonne de silice (éluant : cyclohexane/acétate d'éthyle : 70/30). Le produit est salifié à l'aide d'une solution d'acide chlorhydrique dans l'éthanol.

| *Microanalyse élémentaire :* | | | | |
|---|---|---|---|---|
| | *C %* | *H %* | *N %* | *Cl %* |
| *calculé* | *68,77* | *6,68* | *4,22* | *10,68* |
| *trouvé* | *69,01* | *6,74* | *4,16* | *10,70* |

***Exemple 22** : Trans-2-benzyl-7-méthoxy-1,3,3a,4,9b-pentahydro-(1)-benzopyrano[3,4-c]pyrrole, chlorhydrate*

***Stade A : Trans-1-benzyl-3-hydroxyméthyl-4-(2,4-diméthoxyphényl)pyrrolidine***

Le produit attendu est obtenu selon le procédé décrit au stade A de l'exemple 1 en utilisant le composé décrit lors de la préparation H.

***Stade B : Trans-1-benzyl-3-hydroxyméthyl-4-(2-hydroxy-4-méthoxyphényl)pyrrolidine***

Le produit attendu est obtenu selon le procédé décrit au stade B de l'exemple 1 en utilisant comme produit de départ le composé obtenu au stade précédent.

***Stade C : Trans-2-benzyl-7-méthoxy-1,3,3a,4,9b-pentahydro-(1)-benzopyrano[3,4-c]pyrrole, chlorhydrate***

Le produit attendu est obtenu selon le procédé décrit au stade B de l'exemple 21 à partir du composé obtenu au stade précédent.

| *Microanalyse élémentaire :* | | | | |
|---|---|---|---|---|
| | *C %* | *H %* | *N %* | *Cl %* |
| *calculé* | *68,77* | *6,68* | *4,22* | *10,68* |
| *trouvé* | *68,44* | *6,59* | *4,49* | *10,77* |

***Exemple 23** : Trans-2-benzyl-6-méthoxy-1,3,3a,4,9b-pentahydro-(1)-benzopyrano[3,4-c]pyrrole, chlorhydrate*

Le produit attendu est obtenu selon les stades A, B et C de l'exemple 23 en partant du composé issu de la préparation I.

| *Microanalyse élémentaire :* | | | | |
|---|---|---|---|---|
| | *C %* | *H %* | *N %* | *Cl %* |
| *calculé* | *68,77* | *6,68* | *4,22* | *10,68* |
| *trouvé* | *67,93* | *6,66* | *4,10* | *10,55* |

***Exemple 24** : Cis-2-benzyl-8-chloro-1,3,3a,4,9b-pentahydro-(1)-benzopyrano[3,4-c]pyrrole, chlorhydrate*

***Stade A : Cis-1-benzyl-3-hydroxyméthyl-4-(2-hydroxy-5-chlorophényl)pyrrolidine***

Le produit attendu est obtenu selon le procédé décrit au stade A de l'exemple 12 en utilisant comme produit de départ le composé décrit lors de la préparation J.

***Stade B** : **Cis-2-benzyl-8-chloro-1,3,3a,4,9b-pentahydro-(1)-benzopyrano[3,4-c]pyrrole, chlorhydrate***

Le produit attendu est obtenu selon le procédé décrit au stade B de l'exemple 21 à partir du composé obtenu au stade précédent.

| *Microanalyse élémentaire :* | | | | |
|---|---|---|---|---|
| | *C %* | *H %* | *N %* | *Cl %* |
| *calculé* | *64,29* | *5,70* | *4,17* | *21,09* |
| *trouvé* | *64,82* | *5,82* | *4,19* | *20,81* |

***Exemple 25** : Trans-2-benzyl-8-chloro-1,3,3a,4,9b-pentahydro-(1)-benzopyrano[3,4-c]pyrrole, chlorhydrate*

Le produit attendu est obtenu selon les stades A, B et C de l'exemple 22 en partant du composé issu de la préparation K.

| *Microanalyse élémentaire :* | | | | |
|---|---|---|---|---|
| | *C %* | *H %* | *N %* | *Cl %* |
| *calculé* | *64,29* | *5,70* | *4,17* | *21,09* |
| *trouvé* | *64,01* | *6,01* | *4,22* | *21,05* |

***Exemple 26** : Cis-2-benzyl-6-chloro-1,3,3a,4,9b-pentahydro-(1)-benzopyrano[3,4-c]pyrrole, chlorhydrate*

Le produit attendu est préparé selon les stades A de l'exemple 12 puis B de l'exemple 21 à partir du composé obtenu lors de la préparation L.

| *Microanalyse élémentaire :* | | | | |
|---|---|---|---|---|
| | *C %* | *H %* | *N %* | *Cl %* |
| *calculé* | *64,29* | *5,70* | *4,17* | *21,09* |
| *trouvé* | *63,72* | *5,59* | *4,26* | *24,16* |

***Exemple 27** : Trans-2-benzyl-6-chloro-1,3,3a,4,9b-pentahydro-(1)-benzopyrano[3,4-c]pyrrole, chlorhydrate*

Le produit attendu est obtenu selon les stades A, B et C de l'exemple 1 puis selon le stade D de l'exemple 3 en partant du composé issu de la préparation M.

| Microanalyse élémentaire : | | | | |
|---|---|---|---|---|
| | C % | H % | N % | Cl % |
| calculé | 64,29 | 5,70 | 4,17 | 21,09 |
| trouvé | 64,22 | 5,45 | 4,01 | 21,47 |

***Exemple 28** : Trans-2-benzyl-8-bromo-1,3,3a,4,9b-pentahydro-(1)-benzopyrano[3,4-c]pyrrole, chlorhydrate*

Le produit attendu est obtenu selon les stades A, B et C de l'exemple 1 suivi du stade D de l'exemple 3 en partant du composé issu de la préparation N.

| Microanalyse élémentaire : | | | | | |
|---|---|---|---|---|---|
| | C % | H % | N % | Br % | Cl % |
| calculé | 56,79 | 5,03 | 3,68 | 20,99 | 9,31 |
| trouvé | 57,14 | 5,19 | 3,48 | 20,00 | 9,18 |

***Exemple 29** : Trans-2-benzyl-8-cyano-1,3,3a,4,9b-pentahydro-(1)-benzopyrano[3,4-c]pyrrole, chlorhydrate*

9 mmoles du composé de l'exemple 18 sont mises en solution dans 10,4 ml de diméthylformamide. On ajoute, ensuite, 5 mmoles de cyanure de zinc et 0,3 mmole de tétrakis triphénylphosphine palladium. Le milieu réactionnel est porté à 80°C pendant 6 heures. Après refroidissement, on ajoute 30 ml de toluène et on lave par 2 fois 20 ml d'une solution 2M d'ammoniaque puis, à l'aide d'une solution saturée de chlorure de sodium. Après évaporation des solvants, le produit brut est salifié à l'aide d'une solution d'acide chlorhydrique dans l'éthanol.

| Microanalyse élémentaire : | | | | |
|---|---|---|---|---|
| | C % | H % | N % | Cl % |
| calculé | 69,83 | 5,86 | 8,57 | 10,85 |
| trouvé | 69,41 | 5,77 | 8,53 | 10,83 |

***Exemple 30** : Trans-8-cyano-1,3,3a,4,9b-pentahydro-(1)-benzopyrano[3,4-c]pyrrole, chlorhydrate*

17,2 mmoles du composé obtenu dans l'exemple 29 dans 130 ml d'éthanol et 40 ml d'eau sont hydrogénées pendant 24 heures à 40°C en présence de 500 mg de palladium sur charbon comme catalyseur. Après évaporation des solvants, on obtient le produit attendu.

| Microanalyse élémentaire : | | | | |
|---|---|---|---|---|
| | C % | H % | N % | Cl % |
| calculé | 60,89 | 5,54 | 11,83 | 14,98 |

(suite)

| *Microanalyse élémentaire :* | | | | |
|---|---|---|---|---|
| | *C %* | *H %* | *N %* | *Cl %* |
| *trouvé* | *60,44* | *5,52* | *11,45* | *14,26* |

***Exemple 31** : Trans-2-propyl-8-cyano-1,3,3a,4,9b-pentahydro-(1)-benzopyrano[3,4-c]pyrrole, chlorhydrate*

Le composé obtenu dans l'exemple 30 est traité selon le mode opératoire décrit pour l'exemple 6.

| *Microanalyse élémentaire :* | | | | |
|---|---|---|---|---|
| | *C %* | *H %* | *N %* | *Cl %* |
| *calculé* | *64,63* | *6,87* | *10,05* | *12,72* |
| *trouvé* | *63,93* | *6,78* | *9,53* | *12,21* |

***Exemple 32** : Trans-2-benzyl-1,2,3,3a,4,11c-hexahydro-5-oxa-2-aza-cyclopenta[c]phénantrène, chlorhydrate*

Le produit attendu est obtenu selon les stades A, B et C de l'exemple 1 suivi du stade D de l'exemple 3 en partant du composé issu de la préparation O.

| *Microanalyse élémentaire :* | | | | |
|---|---|---|---|---|
| | *C %* | *H %* | *N %* | *Cl %* |
| *calculé* | *75,10* | *6,30* | *3,98* | *10,08* |
| *trouvé* | *74,51* | *6,25* | *4,16* | *10,02* |

***Exemple 33** : Trans-1,2,3,3a,4,11c-hexahydro-5-oxa-2-aza-cyclopenta[c]phénantrène, chlorhydrate*

Le produit issu de l'exemple 32 est traité selon le mode opératoire de l'exemple 30.

| *Microanalyse élémentaire :* | | | | |
|---|---|---|---|---|
| | *C %* | *H %* | *N %* | *Cl %* |
| *calculé* | *68,83* | *6,16* | *5,35* | *13,54* |
| *trouvé* | *68,49* | *6,25* | *4,99* | *13,47* |

***Exemple 34** : Trans-16-benzyl-12,13,14,15,16,17-hexahydro-11-oxa-16-aza-cyclopenta[a]phénantrène, chlorhydrate*

Le produit attendu est obtenu selon le stade A de l'exemple 1 puis traité selon les modes opératoires décrits aux stades C de l'exemple 2 et B de l'exemple 21 en partant du composé de la préparation P.

| Microanalyse élémentaire : | | | | |
|---|---|---|---|---|
| | C % | H % | N % | Cl % |
| calculé | 75,10 | 6,30 | 3,98 | 10,08 |
| trouvé | 74,89 | 6,30 | 3,93 | 10,19 |

***Exemple 35* : Cis-16-benzyl-12,13,14,15,16,17-hexahydro-11-oxa-16-aza-cyclopenta[a]phénantrène, chlorhydrate**

Le produit attendu est obtenu selon les stades A et B de l'exemple 21 en partant du composé issu de la préparation Q.

| Microanalyse élémentaire : | | | | |
|---|---|---|---|---|
| | C % | H % | N % | Cl % |
| calculé | 75,10 | 6,30 | 3,98 | 10,08 |
| trouvé | 74,53 | 6,38 | 3,85 | 10,02 |

***Exemple 36* : Cis-12,13,14,15,16,17-hexahydro-11-oxa-16-aza-cyclopenta[a]phénantrène, chlorhydrate**

Le produit attendu est obtenu selon le procédé décrit pour l'exemple 30 à partir du composé de l'exemple 35.

| Microanalyse élémentaire : | | | | |
|---|---|---|---|---|
| | C % | H % | N % | Cl % |
| calculé | 68,83 | 6,16 | 5,35 | 13,54 |
| trouvé | 68,41 | 6,22 | 5,56 | 13,46 |

***Exemple 37* : Cis-2-benzyl-1,2,3,3a,4,11c-hexahydro-5-oxa-2-aza-cyclopenta[c]phénantrène, chlorhydrate**

Le produit attendu est obtenu selon les stades A et B de l'exemple 21 en partant du composé issu de la préparation R.

| Microanalyse élémentaire : | | | | |
|---|---|---|---|---|
| | C % | H % | N % | Cl % |
| calculé | 75,10 | 6,30 | 3,98 | 10,08 |
| trouvé | 75,13 | 6,30 | 4,01 | 9,88 |

***Exemple 38* : Cis-1,2,3,3a,4,11c-hexahydro-5-oxa-2-aza-cyclopenta[c]phénantrène, chlorhydrate**

Le produit attendu est obtenu selon le procédé décrit pour l'exemple 30 à partir du composé de l'exemple 37.

| *Microanalyse élémentaire :* | | | | |
|---|---|---|---|---|
| | *C %* | *H %* | *N %* | *Cl %* |
| *calculé* | *68,83* | *6,16* | *5,35* | *13,54* |
| *trouvé* | *68,25* | *6,11* | *5,43* | *13,19* |

**Etude pharmacologique des composés de l'invention**

***Exemple 39*** ***: Mesure de l'affinité in vitro aux récepteurs 5-HT$_{2C}$ et 5-HT$_{2A}$***

**Méthodes**

Les protocoles pour les études de fixation aux récepteurs 5-HT$_{2A}$ et 5-HT$_{2C}$ sont exactement ceux décrits par H. Canton et al. (Eur. J. Pharmacol., 191, 93, 1990) et M.J. Millan et al. (J. Pharmacol. Exp. Ther., 262, 451-463, 1992). Pour le 5-HT$_{2A}$ : cortex frontal du rat/[$^3$H]-kétansérine (1.0 nM). Pour 5-HT$_{2C}$ : plexus cérébral du cochon/[$^3$H]-mesulergine (1.0 nM).

Les Doses Inhibitrices$_{50}$ (DI$_{50}$) sont déterminées par une analyse de régression et les pKis calculés comme suit :

$$Ki = \frac{IC_{50}}{1 + [L] / Kd}$$

[L] = concentration du Kd - Kd = constante de dissociation

**Résultats**

les résultats obtenus avec les composés de référence et les composés de l'invention sont regroupés dans le tableau ci-dessous.

Le SB 200,646 a montré une modeste affinité pour les récepteurs 5-HT$_{2C}$ de l'ordre de 200 nM et n'a présenté qu'une faible affinité pour les sites 5-HT$_{2A}$. Sa sélectivité est alors de 6 pour les sites 5-HT$_{2C}$. En revanche, le MDL 100,907 a montré une très importante sélectivité de l'ordre de 200 pour les récepteurs 5-HT$_{2A}$ pour lesquels il a une très forte affinité.

Les composés de l'invention ont une meilleure affinité pour les récepteurs 5-HT$_{2C}$ que le composé de référence SB 200,646. De plus, ils présentent une sélectivité meilleure pour les récepteurs 5-HT$_{2C}$ par rapport aux récepteurs 5-HT$_{2A}$.

On peut citer, plus particulièrement, les composés des exemples 19 et 20 qui sont respectivement 9 et 14 fois plus puissants que le composé de référence SB 200,646 ainsi que les composés des exemples 6 et 7 qui présentent une sélectivité 2 fois supérieure à celle du composé de référence SB 200,646.

| Composé | 5-HT$_{2C}$ Ki (nM) | 5-HT$_{2A}$ Ki (nM) | Rapport des affinités 5-HT$_{2A}$/5-HT$_{2C}$ |
|---|---|---|---|
| Exemple 2 | 195 | 490 | 2.5 |
| Exemple 3 | 48 | 269 | 5.6 |
| Exemple 4 | 431 | 3468 | 8.0 |
| Exemple 6 | 104 | 1738 | 16.7 |
| Exemple 7 | 120 | 1820 | 15.2 |

(suite)

| Composé | 5-$HT_{2C}$ Ki (nM) | 5-$HT_{2A}$ Ki (nM) | Rapport des affinités 5-$HT_{2A}$/5-$HT_{2C}$ |
|---|---|---|---|
| Exemple 8 | 107 | 95 | 0.9 |
| Exemple 9 | 468 | 1097 | 2.3 |
| Exemple 10 | 2188 | > 10000 | > 4.8 |
| Exemple 11 | 4074 | > 10000 | > 2.3 |
| Exemple 12 | 832 | 3549 | 4.3 |
| Exemple 13 | 3090 | > 10000 | > 3.33 |
| Exemple 14 | 218.8 | 1096 | 5.00 |
| Exemple 15 | 36.3 | 389.0 | 10.7 |
| Exemple 16 | 263 | 3311 | 12.6 |
| Exemple 18 | 871 | 1867 | 2.1 |
| Exemple 19 | 22 | 155 | 7.0 |
| Exemple 20 | 14.4 | 102 | 7.1 |
| Exemple 21 | 871 | 617 | 0.7 |
| Exemple 22 | 478 | 170 | 3.6 |
| Exemple 24 | 38 | 107 | 2.8 |
| Exemple 33 | 51 | 107 | 2.1 |
| Exemple 34 | 776 | 1479 | 1.9 |
| Exemple 35 | 331 | 955 | 2.9 |
| Exemple 36 | 25.7 | 257 | 10 |
| Exemple 37 | 155 | 1349 | 8.7 |
| Exemple 38 | 22.9 | 97.7 | 4.3 |
| SB 200,646 | 204.0 | 1318 | 6.5 |
| MDL 100,907 | 107.2 | 0.59 | 0.006 |

Chaque valeur représente la moyenne de deux à quatre déterminations.

***Exemple 40** : Composition pharmaceutique*

Formule de préparation pour 1000 comprimés dosés à 10 mg :

| | |
|---|---|
| Composé de l'exemple 16 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I)

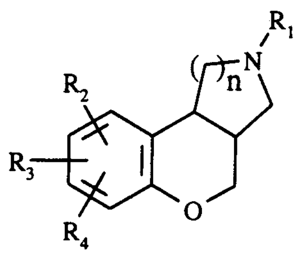

(I)

: dans laquelle :

n représente 1 ou 2,

$R_1$ représente un atome d'hydrogène ou un groupement alkyle ($C_1$-$C_6$) linéaire ou ramifié, benzyle, acétyle, benzoyle, allyle, pyridinecarbonyle, pyridineméthyle, pyridineaminocarbonyle, phtalimidoalkyle ($C_1$-$C_6$) linéaire ou ramifié, (thiochroman-8-yloxy)alkyle ($C_1$-$C_4$) linéaire ou ramifié, (benzodioxanyloxy) alkyle ($C_1$-$C_4$) linéaire ou ramifié ou un groupement acylaminoalkyle ($C_1$-$C_6$) linéaire ou ramifié (dans lequel le groupement acyle est un groupement benzoyle, naphtylcarbonyle, thiénylcarbonyle, alkylcarbonyle ($C_1$-$C_6$) linéaire ou ramifié, furylcarbonyle, pyrrolylcarbonyle, pyridinylcarbonyle, cycloalkyle ($C_3$-$C_7$) carbonyle, chacun de ces groupements étant éventuellement substitué par un ou plusieurs atomes d'halogène ou groupements trihalogénométhyle, alkoxy ou hydroxy),

$R_2$, $R_3$ ou $R_4$, identiques ou différents représentent un atome d'hydrogène, d'halogène ou un groupement alkyle ($C_1$-$C_6$) linéaire ou ramifié, alkoxy ($C_1$-$C_6$) linéaire ou ramifié, hydroxy, acétyle, aminocarbonyle, aminométhyle, cyano, nitro, amino, phényle (substitué ou non par un ou plusieurs atomes d'halogène ou groupements hydroxy, alkoxy ($C_1$-$C_6$) linéaire ou ramifié, alkyle ($C_1$-$C_6$) linéaire ou ramifié ou trihalogénométhyle), furyle, pyridinyle, thiényle ou pyrrolyle,
ou bien,
$R_2$ et $R_3$, lorsqu'ils sont situés sur des carbones adjacents, forment avec les atomes de carbone qui les portent, un noyau furane ou phényle,

leurs isomères ainsi que leurs sels d'addition à un acide pharmaceutiquement acceptable.

2. Composé de formule (I) selon la revendication 1 dans laquelle n est égal à 1, leurs isomères ainsi que leurs sels d'addition à un acide pharmaceutiquement acceptable.

3. Composé de formule (I) selon la revendication 1 dans laquelle $R_1$ représente un groupement benzyle, leurs isomères ainsi que leurs sels d'addition à un acide pharmaceutiquement acceptable.

4. Composé de formule (I) selon la revendication 1 dans laquelle l'un au moins des groupements $R_2$, $R_3$ ou $R_4$ représente un groupement alkoxy ($C_1$-$C_6$) linéaire ou ramifié ou un groupement hydroxy, leurs isomères ainsi que leurs sels d'addition à un acide pharmaceutiquement acceptable.

5. Procédé de préparation des composés de formule (I) selon la revendication 1 dans laquelle n = 1, caractérisé en ce que l'on utilise comme produit de départ une pyrrolidine de formule (II), sous forme de couple d'énantiomères, ou d'énantiomère pur :

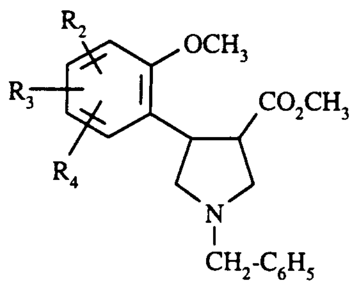

(II)

dans laquelle $R_2$, $R_3$ et $R_4$ sont tels que définis dans la formule (I),
composé de formule (II), qui, lorsqu'il est sous la forme d'un couple d'énantiomères,
que l'on fait réagir avec de l'hydrure de lithium aluminium dans un solvant inerte pour conduire à la pyrrolidine de formule (III) :

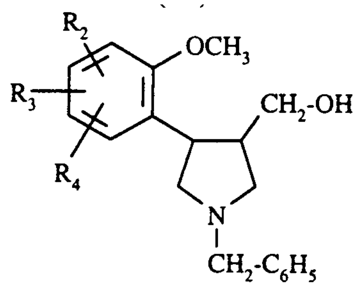

(III)

dans laquelle $R_2$, $R_3$ et $R_4$ ont la même signification que dans la formule (I),
dont on transforme la fonction méthoxy en fonction hydroxy par réaction en présence d'éthanethiolate de sodium ou de tribromure de bore, puis que l'on fait réagir avec de l'acide chlorhydrique gazeux en présence de chlorure de thionyle, en milieu chloroformique, pour conduire au composé de formule (IV) :

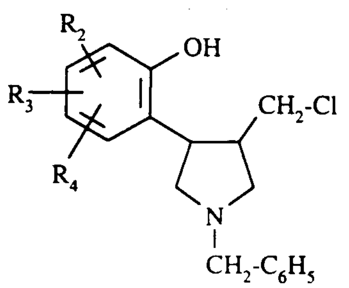

(IV)

dans laquelle $R_2$, $R_3$ et $R_4$ ont la même signification que dans la formule (I),
qui est alors cyclisé en milieu basique,
pour conduire au composé de formule (I/a), cas particulier des composés de formule (I) :

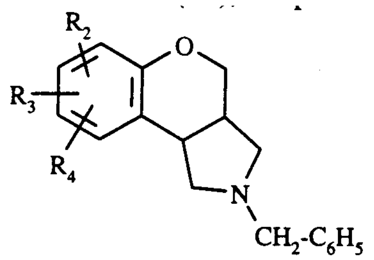

(I/a)

dans laquelle $R_2$, $R_3$ ou $R_4$ ont la même signification que dans la formule (I),
composé de formule (I/a), dont on peut déprotéger, si on le souhaite, la fonction amine par hydrogénation catalytique,
pour conduire au composé de formule (I/b), cas particulier des composés de formule (I) :

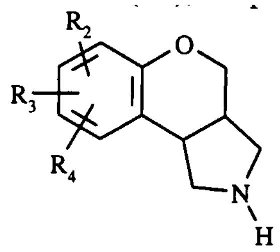

(I/b)

dans laquelle $R_2$, $R_3$ et $R_4$ ont la même signification que dans la formule (I), composé de formule (I/b), que l'on peut faire réagir, si on le souhaite, avec un dérivé halogéné :

$$R'_1X$$

dans laquelle :

- X représente un atome d'halogène et
- $R'_1$ représente un groupement alkyle ($C_1$-$C_6$) linéaire ou ramifié, acétyle, benzoyle, pyridinecarbonyle, pyridineméthyle, 3-pyridineaminocarbonyle, phtalimidoalkyle ($C_1$-$C_6$) linéaire ou ramifié,

pour conduire

- soit au composé de formule (I/c), cas particulier des composés de formule (I) :

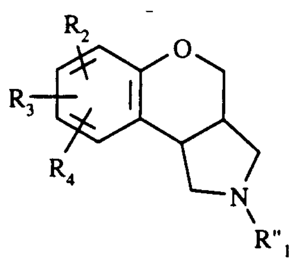

(I/c)

dans laquelle $R_2$, $R_3$ et $R_4$ ont la même signification que dans la formule (I) et $R''_1$ représente un groupement alkyle ($C_1$-$C_6$) linéaire ou ramifié, acétyle, benzoyle, pyridinecarbonyle, pyridineméthyle, 3-pyridineaminocarbonyle ou phtalimidoalkyle ($C_1$-$C_6$) linéaire ou ramifié,
- soit au composé de formule

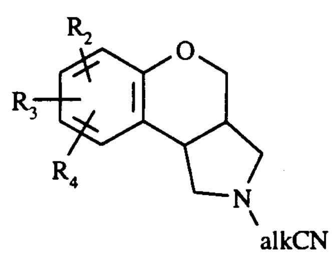

(V)

(V) : dans laquelle $R_2$, $R_3$ et $R_4$ ont la même signification que dans la formule (I) et alkCN représente un groupement cyanoalkyle ($C_1$-$C_6$) linéaire ou ramifié,
composé de formule (V) dont on réduit le groupement cyano en groupement amino et que l'on fait réagir sur un halogénure de benzoyle (substitué éventuellement par un atome d'halogène), pour conduire au composé de formule (I/d), cas particulier des composés de formule (I) :

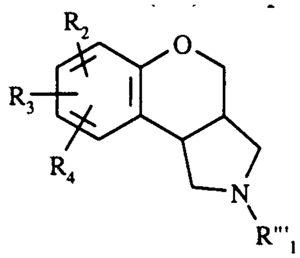

(I/d)

dans laquelle $R_2$, $R_3$ et $R_4$ ont la même signification que dans la formule (I) et $R'''_1$ représente un groupement benzoylaminoalkyle ($C_1$-$C_6$) linéaire ou ramifié (substitué éventuellement sur le noyau phényle par un atome d'halogène),

composé de formule (I/a), (I/b), (I/c) ou (I/d) :

- que l'on purifie éventuellement selon une technique classique de purification,
- dont on sépare, si on le souhaite, les énantiomères selon une technique classique de séparation,
- et que l'on transforme, le cas échéant, en ses sels d'addition à un acide pharmaceutiquement acceptable,
- étant entendu que les substituants $R_2$, $R_3$ et $R_4$ peuvent être introduits ou modifiés tout au long de la synthèse des composés de formule (I), selon des techniques classiques de la chimie organique.

**6.** Procédé de préparation des composés de formule (I) selon la revendication 1 dans laquelle n = 2, caractérisé en ce que l'on utilise comme produit de départ le composé de formule (IX) :

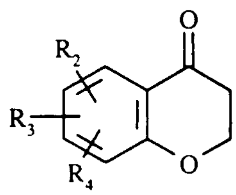

(IX)

dans laquelle $R_2$, $R_3$ et $R_4$ sont tels que définis dans la formule (I), que l'on fait réagir avec le bromure de méthylmagnésium puis avec l'acide p.toluènesulfonique, pour conduire au composé de formule (X) :

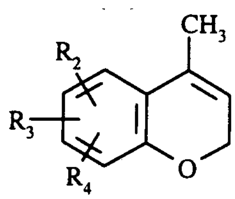

(X)

dans laquelle $R_2$, $R_3$ et $R_4$ ont la même signification que dans la formule (I), que l'on met en réaction avec la benzylamine en présence de formaldéhyde,

pour conduire au composé de formule (XI) :

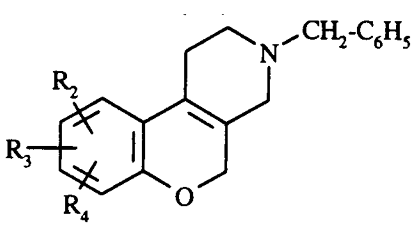

(XI)

dans laquelle $R_2$, $R_3$ et $R_4$ ont la même signification que dans la formule (I),

qui subit alors une hydrogénation catalytique, pour conduire au composé de formule (I/e), cas particulier des composés de formule (I) :

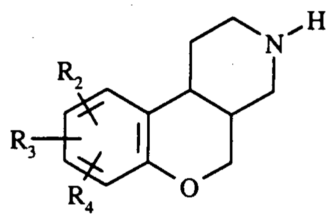

(I/e)

dans laquelle $R_2$, $R_3$ et $R_4$ ont la même signification que dans la formule (I), composé de formule (I/e), que l'on peut faire réagir, si on le souhaite, avec un dérivé halogéné de formule (XII) :

$$R'_{1a}\text{-}X \qquad (XII)$$

dans laquelle :

X représente un atome d'halogène et
$R'_{1a}$ représente un groupement alkyle ($C_1$-$C_6$) linéaire ou ramifié, acétyle, benzoyle, benzyle, pyridinecarbonyle, pyridineméthyle, 3-pyridineaminocarbonyle, phtalimidoalkyle ($C_1$-$C_6$) linéaire ou ramifié,

pour conduire

- soit au composé de formule (I/f) cas particulier des composés de formule (I) :

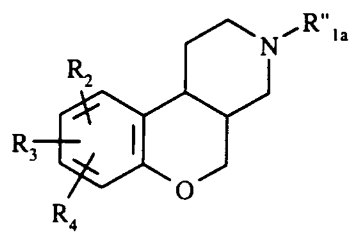

(I/f)

dans laquelle $R_2$, $R_3$ et $R_4$ ont la même signification que dans la formule (I) et $R''_{1a}$ représente un groupement alkyle ($C_1$-$C_6$) linéaire ou ramifié, acétyle, benzoyle, benzyle, pyridinecarbonyle, pyridineméthyle, 3-pyridineaminocarbonyle ou phtalimidoalkyle ($C_1$-$C_6$) linéaire ou ramifié,
- soit au composé de formule (XIII) :

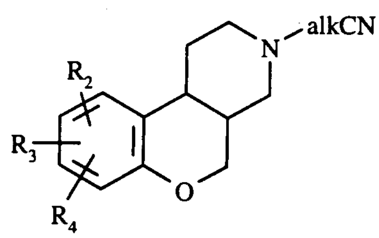

(XIII)

dans laquelle $R_2$, $R_3$ et $R_4$ ont la même signification que dans la formule (I) et alkCN représente un groupement cyanoalkyle ($C_1$-$C_6$) linéaire ou ramifié,
composé de formule (XIII) dont on réduit le groupement cyano en groupement amino et que l'on fait réagir sur un halogénure de benzoyle (substitué éventuellement par un atome d'halogène),
pour conduire au composé de formule (I/g), cas particulier des composés de formule (I) :

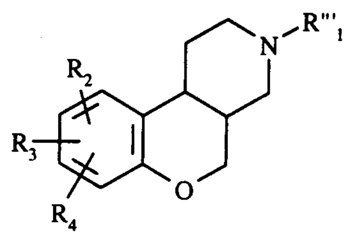

(I/g)

dans laquelle $R_2$, $R_3$ et $R_4$ ont la même signification que dans la formule (I) et $R'''_1$ représente un groupement benzoylaminoalkyle ($C_1$-$C_6$) linéaire ou ramifié (substitué éventuellement sur le noyau phényle par un atome d'halogène),

composé de formule (I/e), (I/f) ou (I/g) :

- que l'on purifie éventuellement selon une technique classique de purification,
- dont on sépare, si on le souhaite, les énantiomères selon une technique classique de séparation,
- et que l'on transforme, le cas échéant, en ses sels d'addition à un acide pharmaceutiquement acceptable,
- étant entendu que les substituants $R_2$, $R_3$ et $R_4$ peuvent être introduits ou modifiés tout au long de la synthèse des composés de formule (I), selon des techniques classiques de la chimie organique.

7. Compositions pharmaceutiques contenant comme principe actif au moins un composé selon l'une quelconques des revendications 1 à 4 seul ou en combinaison avec un ou plusieurs véhicules inertes, non toxiques pharmaceutiquement acceptables.

8. Compositions pharmaceutiques selon la revendication 7 pour utilisation dans le traitement des maladies nécessitant un ligand aux récepteurs 5-HT2C comme l'anxiété, la dépression, les troubles impulsifs, la schizophrénie, les troubles de l'appétit, les maladies cardiovasculaires, le dysfonctionnement sexuel, l'ischémie cérébrale, l'abus de drogues, les troubles du sommeil et la migraine.

## Claims

1. Compound of formula (I):

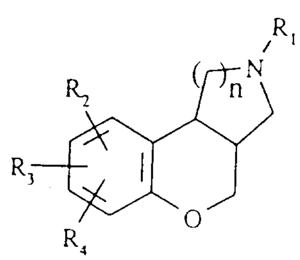

(I)

wherein:

| | |
|---|---|
| n | represents 1 or 2, |
| $R_1$ | represents a hydrogen atom or a linear or branched ($C_1$-$C_6$)alkyl group, a benzyl group, an acetyl group, a benzoyl group, an allyl group, a pyridinecarbonyl group, a pyridinemethyl group, a pyridineaminocarbonyl group, a phthalimido-($C_1$-$C_6$)alkyl group in which the alkyl moiety is linear or branched, a thiochroman-8-yloxy-($C_1$-$C_4$)alkyl group in which the alkyl moiety is linear or branched, a benzodioxanyloxy-($C_1$-$C_4$)alkyl group in which the alkyl moiety is linear or branched or an acylamino-($C_1$-$C_6$)alkyl group in which the alkyl moiety is linear or branched (in which the acyl group is a benzoyl, naphthylcarbonyl, thienylcarbonyl, linear or branched ($C_1$-$C_6$)alkylcarbonyl, furylcarbonyl, pyrrolylcarbonyl, pyridinylcarbonyl or ($C_3$-$C_7$)cycloalkylcarbonyl group, each of those groups being optionally substituted by one or more halogen atoms or trihalomethyl, alkoxy or hydroxy groups), |
| $R_2$, $R_3$ and $R_4$, | which may be the same or different, represent a hydrogen or halogen atom or a linear or branched ($C_1$-$C_6$)alkyl group, a linear or branched ($C_1$-$C_6$)alkoxy group, a hydroxy group, an |

acetyl group, an aminocarbonyl group, an aminomethyl group, a cyano group, a nitro group, an amino group, a phenyl group (unsubstituted or substituted by one or more halogen atoms or hydroxy, linear or branched ($C_1$-$C_6$)alkoxy, linear or branched ($C_1$-$C_6$)alkyl or trihalomethyl groups), a fury group, a pyridinyl group, a thienyl group or a pyrrolyl group,
or
$R_2$ and $R_3$, when they are located on adjacent carbon atoms, form with the carbon atoms carrying them a furan or phenyl ring,

their isomers and addition salts thereof with a pharmaceutically acceptable acid.

2. Compound of formula (I) according to claim 1 wherein n is 1, their isomers and addition salts thereof with a pharmaceutically acceptable acid.

3. Compound of formula (I) according to claim 1 wherein $R_1$ represents a benzyl group, their isomers and addition salts thereof with a pharmaceutically acceptable acid.

4. Compound of formula (I) according to claim 1 wherein at least one of the groups $R_2$, $R_3$ and $R_4$ represents a linear or branched ($C_1$-$C_6$)alkoxy group or a hydroxy group, their isomers and addition salts thereof with a pharmaceutically acceptable acid.

5. Process for the preparation of compounds of formula (I) according to claim 1 wherein n = 1, characterised in that there is used as starting material a pyrrolidine of formula (II), in the form of a pair of enantiomers or of a pure enantiomer:

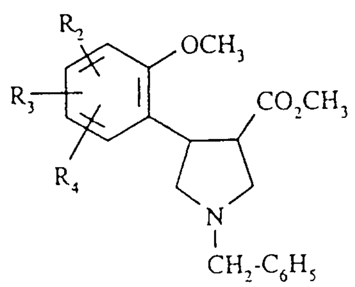

(II)

wherein $R_2$, $R_3$ and $R_4$ are as defined for formula (I),
which compound of formula (II), when it is in the form of a pair of enantiomers, is reacted with lithium aluminium hydride in an inert solvent to yield a pyrrolidine of formula (III):

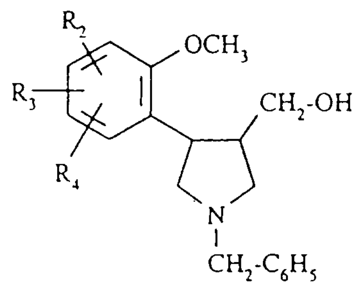

(III)

wherein $R_2$, $R_3$ and $R_4$ are as defined for formula (I),
the methoxy function of which is converted to the hydroxy function by reaction in the presence of sodium ethanethiolate or of boron tribromide, followed by reaction with gaseous hydrogen chloride in the presence of thionyl chloride, in a chloroform medium,
to yield a compound of formula (IV):

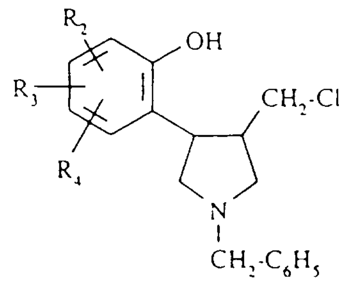

(IV)

wherein $R_2$, $R_3$ and $R_4$ are as defined for formula (I), which is then cyclised in a basic medium, to yield a compound of formula (I/a), a particular case of the compounds of formula (I):

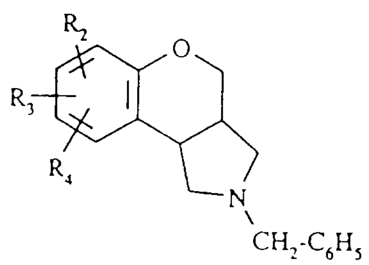

(I/a)

wherein $R_2$, $R_3$ and $R_4$ are as defined for formula (I), the amino function of which compound of formula (I/a) may, if desired, be deprotected by catalytic hydrogenation, to yield a compound of formula (I/b), a particular case of the compounds of formula (I):

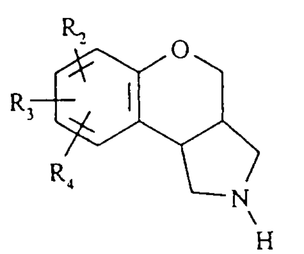

(I/b)

wherein $R_2$, $R_3$ and $R_4$ are as defined for formula (I), which compound of formula (I/b) may, if desired, be reacted with a halogenated compound:

$$R'_1X$$

wherein:

X represents a halogen atom, and

$R'_1$ represents a linear or branched ($C_1$-$C_6$)alkyl group, an acetyl group, a benzoyl group, a pyridinecarbonyl group, a pyridinemethyl group, a 3-pyridineaminocarbonyl group or a phthalimido-($C_1$-$C_6$)alkyl group in which the alkyl moiety is linear or branched,

to yield

- a compound of formula (I/c), a particular case of the compounds of formula (I):

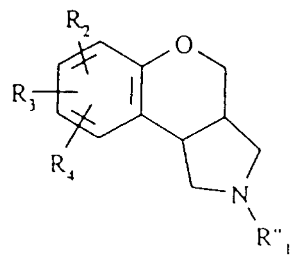

(I/c)

wherein $R_2$, $R_3$ and $R_4$ are as defined for formula (I) and $R''_1$ represents a linear or branched ($C_1$-$C_6$)alkyl group, an acetyl group, a benzoyl group, a pyridinecarbonyl group, a pyridinemethyl group, a 3-pyridineaminocarbonyl group or a phthalimido-($C_1$-$C_6$)alkyl group in which the alkyl moiety is linear or branched,

- or a compound of formula (V):

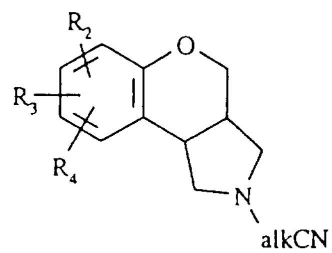

(V)

wherein $R_2$, $R_3$ and $R_4$ are as defined for formula (I) and alkCN represents a cyano-($C_1$-$C_6$)alkyl group in which the alkyl moiety is linear or branched,
the cyano group of which compound of formula (V) is reduced to an amino group, followed by reaction with a benzoyl halide (optionally substituted by a halogen atom), to yield a compound of formula (I/d), a particular case of the compounds of formula (I):

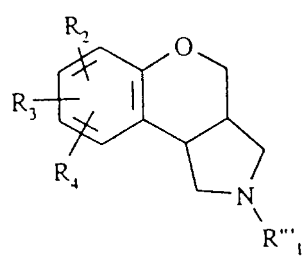

(I/d)

wherein $R_2$, $R_3$ and $R_4$ are as defined for formula (I) and $R'''_1$ represents a benzoylamino-($C_1$-$C_6$)alkyl group in which the alkyl moiety is linear or branched (optionally substituted on the phenyl ring by a halogen atom),
which compound of formula (I/a), (I/b), (I/c) or (I/d):

- is purified, if necessary, according to a conventional purification technique,
- separated, if desired, into its enantiomers according to a conventional separation technique, and
- converted, where appropriate, into addition salts thereof with a pharmaceutically acceptable acid,
- it being understood that the substituents $R_2$, $R_3$ and $R_4$ can be introduced or modified throughout the synthesis of the compounds of formula (I), according to conventional techniques of organic chemistry.

**6.** Process for the preparation of compounds of formula (I) according to claim 1 wherein n = 2, characterised in that there is used as starting material a compound of formula (IX):

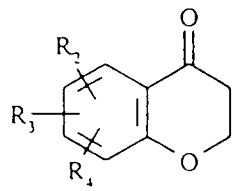

(IX)

wherein $R_2$, $R_3$ and $R_4$ are as defined for formula (I), which is reacted with methylmagnesium bromide and then with para-toluenesulphonic acid,
to yield a compound of formula (X):

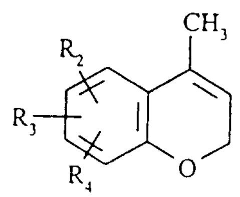

(X)

wherein $R_2$, $R_3$ and $R_4$ are as defined for formula (I), which is reacted with benzylamine in the presence of formaldehyde,
to yield a compound of formula (XI):

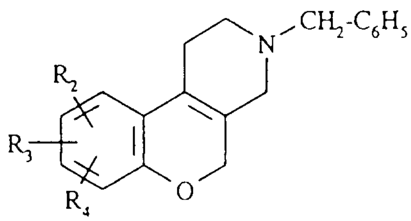

(XI)

wherein $R_2$, $R_3$ and $R_4$ are as defined for formula (I),
which is then subjected to catalytic hydrogenation, to yield a compound of formula (I/e), a particular case of the compounds of formula (I):

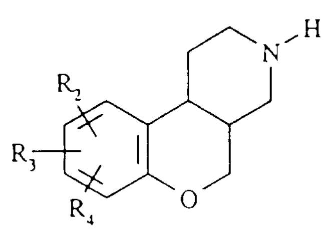

(I/e)

wherein $R_2$, $R_3$ and $R_4$ are as defined for formula (I),
which compound of formula (I/e) may be reacted, if desired, with a halogenated compound of formula (XII):

$$R'_{1a}\text{-}X \qquad (XII)$$

wherein:

X represents a halogen atom, and
$R'_{1a}$ represents a linear or branched ($C_1$-$C_6$)alkyl group, an acetyl group, a benzoyl group, a benzyl group, a

pyridinecarbonyl group, a pyridinemethyl group, a 3-pyridineaminocarbonyl group or a phthalimido-($C_1$-$C_6$)alkyl group in which the alkyl moiety is linear or branched,

to yield

- a compound of formula (I/f), a particular case of the compounds of formula (I):

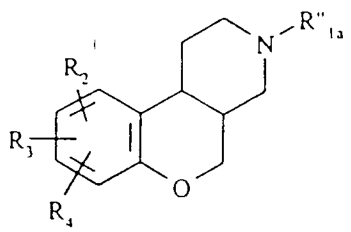

(I/f)

wherein $R_2$, $R_3$ and $R_4$ are as defined for formula (I) and $R''_{1a}$ represents a linear or branched ($C_1$-$C_6$)alkyl group, an acetyl group, a benzoyl group, a benzyl group, a pyridinecarbonyl group, a pyridinemethyl group, a 3-pyridineaminocarbonyl group or a phthalimido-($C_1$-$C_6$)alkyl group in which the alkyl moiety is linear or branched,

- or a compound of formula (XIII):

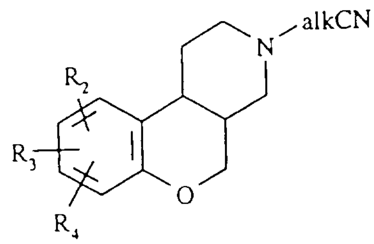

(XIII)

wherein $R_2$, $R_3$ and $R_4$ are as defined for formula (I) and alkCN represents a cyano-($C_1$-$C_6$)alkyl group in which the alkyl moiety is linear or branched, the cyano group of which compound of formula (XIII) is reduced to an amino group, followed by reaction with a benzoyl halide (optionally substituted by a halogen atom), to yield a compound of formula (I/g), a particular case of the compounds of formula (I):

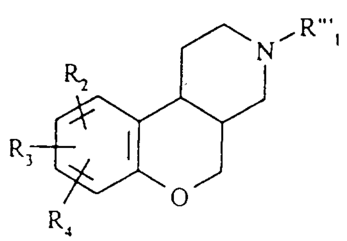

(I/g)

wherein $R_2$, $R_3$ and $R_4$ are as defined for formula (I) and $R'''_1$ represents a benzoylamino-($C_1$-$C_6$)alkyl group in which the alkyl moiety is linear or branched (optionally substituted on the phenyl ring by a halogen atom), which compound of formula (I/e), (I/f) or (I/g):

- is purified, if necessary, according to a conventional purification technique,
- separated, if desired, into its enantiomers according to a conventional separation technique, and
- converted, where appropriate, into addition salts thereof with a pharmaceutically acceptable acid,
- it being understood that the substituents $R_2$, $R_4$ and $R_4$ can be introduced or modified throughout the synthesis of the compounds of formula (I), according to conventional techniques of organic chemistry.

7. Pharmaceutical compositions comprising as active ingredient at least one compound according to any one of claims 1 to 4 on its own or in combination with one or more inert, non-toxic pharmaceutically acceptable carriers.

8. Pharmaceutical compositions according to claim 7 for use in the treatment of diseases requiring a 5-HT2C receptor ligand, such as anxiety, depression, impulsive disorders, schizophrenia, appetite disorders, cardiovascular diseases, sexual dysfunction, cerebral ischemia, drug abuse, sleep disorders and migraine.

**Patentansprüche**

1. Verbindung der Formel (I):

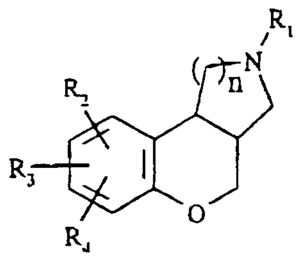

(I)

in der

| | |
|---|---|
| n | 1 oder 2, |
| $R_1$ | ein Wasserstoffatom oder eine geradkettige oder verzweigte ($C_1$-$C_6$)-Alkylgruppe, Benzylgruppe, Acetylgruppe, Benzoylgruppe, Allylgruppe, Pyridincarbonylgruppe, Pyridinmethylgruppe, Pyridinaminocarbonylgruppe, Phthalimido-($C_1$-$C_6$)-alkylgruppe mit geradkettiger oder verzweigter Alkylgruppe, (Thiochroman-8-yloxy)-($C_1$-$C_4$)-alkylgruppe mit geradkettiger oder verzweigter Alkylgruppe, (Benzodioxanyloxy)-($C_1$-$C_4$)-alkylgruppe mit geradkettiger oder verzweigter Alkylgruppe oder Acylamino-($C_1$-$C_6$)-alkylgruppe mit geradkettiger oder verzweigter Alkylgruppe (worin die Acylgruppe eine Benzoylgruppe, Naphthylcarbonylgruppe, Thienylcarbonylgruppe, geradkettige oder verzweigte ($C_1$-$C_6$)-Alkylcarbonylgruppe, Furylcarbonylgruppe, Pyrrolylcarbonylgruppe, Pyridinylcarbonylgruppe, ($C_3$-$C_7$)-Cycloalkylcarbonylgruppe, wobei jede dieser Gruppen gegebenenfalls durch ein oder mehrere Halogenatome oder Trihalogenmethylgruppen, Alkoxygruppen oder Hydroxygruppe substituiert ist), |
| $R_2$, $R_3$ oder $R_4$, | die gleichartig oder verschieden sein können, jeweils ein Wasserstoffatom, ein Halogenatom oder eine geradkettige oder verzweigte ($C_1$-$C_6$)-Alkylgruppe, geradkettige oder verzweigte ($C_1$-$C_6$)-Alkoxygruppe, Hydroxygruppe, Acetylgruppe, Aminocarbonylgruppe, Aminomethylgrupe, Cyanogruppe, Nitrogruppe, Aminogruppe, Phenylgruppe (die gegebenenfalls durch ein oder mehrere Halogenatome oder Hydroxygruppen, geradkettige oder verzweigte ($C_1$-$C_6$)-Alkoxygruppen, geradkettige oder verzweigte ($C_1$-$C_6$)-Alkylgruppen oder Trihalogenmethylgruppen substituiert ist), Furylgruppe, Pyridinylgruppe, Thienylgruppe oder Pyrrolylgruppe<br>oder<br>$R_2$ und $R_3$, wenn sie an benachbarten Kohlenstoffatomen vorliegen, zusammen mit den sie tragenden Kohlenstoffatomen einen Furan-oder Phenylkern bedeuten, |

deren Isomere sowie deren Additionssalze mit einer pharmazeutisch annehmbaren Säuren.

2. Verbindung der Formel (I) nach Anspruch 1, worin n den Wert 1 besitzt, deren Isomere sowie deren Additionssalze mit einer pharmazeutisch annehmbaren Säure.

3. Verbindung der Formel (I) nach Anspruch 1, worin $R_1$ eine Benzylgruppe darstellt, deren Isomere sowie deren Additionssalze mit einer pharmazeutisch annehmbaren Säure.

4. Verbindung der Formel (I) nach Anspruch 1, in der mindestens eine der Gruppen $R_2$, $R_3$ oder $R_4$ eine geradkettige oder verzweigte ($C_1$-$C_6$)-Alkoxygruppe oder eine Hydroxygruppe darstellt, deren Isomere sowie deren Additionssalze mit einer pharmazeutisch annehmbaren Säure.

5. Verfahren zur Herstellung der Verbindungen der Formel (I) nach Anspruch 1, in der n den Wert 1 besitzt, dadurch gekennzeichnet, daß man als Ausgangsprodukt ein Pyrrolidin der Formel (II):

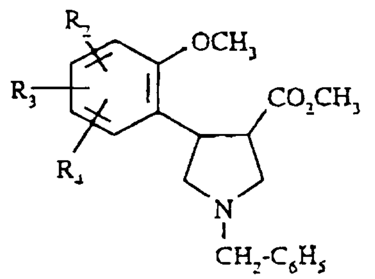

(II)

in der $R_2$, $R_3$ und $R_4$ die bezüglich der Formel (I) angegebenen Bedeutungen besitzen,
in Form des Enantiomerenpaars oder des reinen Enantiomeren verwendet, welche Verbindung der Formel (II), wenn sie in Form eines Enantiomerenpaars vorliegt, mil Lithiumaluminiumhydrid in einem inerten Lösungsmittel umgesetzt wird zur Bildung des Pyrrolidins der Formel (III):

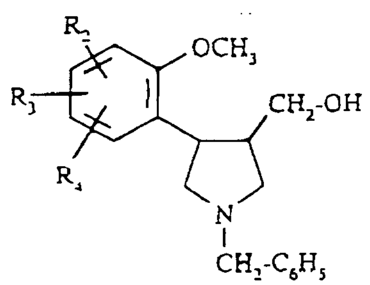

(III)

in der $R_2$, $R_3$ und $R_4$ die bezüglich der Formel (I) angegebenen Bedeutungen besitzen,
dessen Methoxygruppe man durch Reaktion in Gegenwart von Natriumethanthiolat oder Bortribromid in die Hydroxyfunktion umwandelt und dann mit gasförmiger Chlorwasserstoffsäure in Gegenwart von Thionylchlorid in Chloroformmedium umsetzt zur Bildung der Verbindung der Formel (IV):

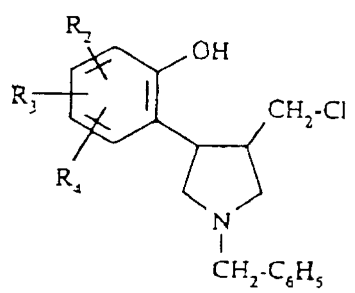

(IV)

in der $R_2$, $R_3$ und $R_4$ die bezüglich der Formel (I) angegebenen Bedeutungen besitzen,
welche anschließend in basischem Medium cyclisiert wird zur Bildung der Verbindung der Formel (I/a), einem Sonderfall der Verbindungen der Formel (I);

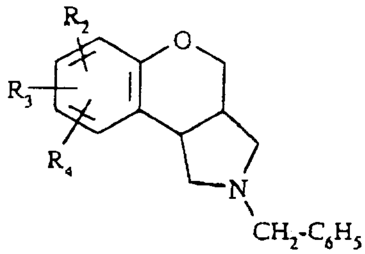

(I/a)

in der $R_2$, $R_3$ und $R_4$ die bezüglich der Formel (I) angegebenen Bedeutungen besitzen, welche Verbindung der Formel (I/a) man gewünschtenfalls in die Schutzgruppe der Aminfunktion durch katalytische Hydrierung abspalten kann, zur Bildung der Verbindung der Formel (I/b), einem Sonderfall der Verbindungen der Formel (I):

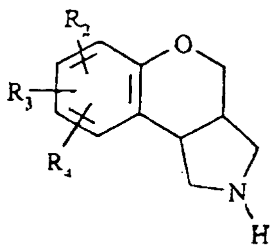

(I/b)

in der $R_2$, $R_3$ und $R_4$ die bezüglich der Formel (I) angegebenen Bedeutungen besitzen, welche Verbindung der Formel (I/b) man gewünschtenfalls mit einem Halogenderi vat der Formel:

$$R'_1X$$

in der:

X ein Halogenatom und
$R'_1$ eine geradkettige oder verzweigte ($C_1$-$C_6$)-Alkylgruppe, Acetylgruppe, Benzoylgruppe, Pyridincarbonylgruppe, Pyridinmethylgruppe, 3-Pyridinaminocarbonylgruppe, Phthalimido-($C_1$-$C_6$)-alkylgruppe mit geradkettiger oder verzweigter Alkylgruppe bedeuten

umsetzt zur Bildung

- entweder der Verbindung der Formel (I/c), einem Sonderfall der Verbindungen der Formel (I):

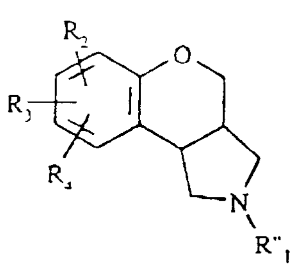

(I/c)

in der $R_2$, $R_3$ und $R_4$ die bezüglich der Formel (I) angegebenen Bedeutungen besitzen und $R''_1$ eine geradkettige oder verzweigte ($C_1$-$C_6$)-Alkylgruppe, Acetylgruppe, Benzoylgruppe, Pyridincarbonylgruppe, Pyridinmethylgruppe, 3-Pyridinaminocarbonylgruppe oder Phthalimido-($C_1$-$C_6$)-alkylgruppe mit geradkettiger oder

verzweigter Alkylgruppe bedeutet,

- oder der Verbindung der Formel (V):

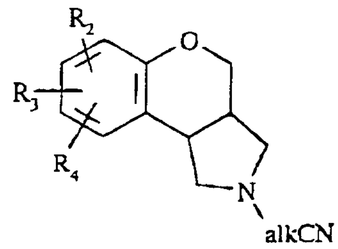

(V)

in der $R_2$, $R_3$ und $R_4$ die bezüglich der Formel (I) angegebenen Bedeutungen besitzen und alkCN eine geradkettige oder verzweigte Cyan-($C_1$-$C_6$)-alkylgruppe darstellt,
wonach man die Cyanogruppe der Verbindung der Formel (V) zu der Aminogruppe reduziert und mit einem (gegebenenfalls durch ein Halogenatom substituierten) Benzoylhalogenid umsetzt zur Bildung der Verbindung der Formel (I/d), einem Sonderfall der Verbindungen der Formel (I):

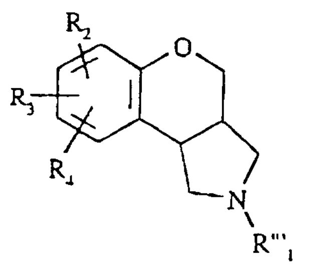

(I/d)

in der $R_2$, $R_3$ und $R_4$ die bezüglich der Formel (I) angegebenen Bedeutungen besitzen und $R'''_1$ eine (gegebenenfalls am Phenylkern durch ein Halogenatom substituierte) Benzoylamino-($C_1$-$C_6$)-alkylgruppe mit geradkettiger oder verzweigter Alkylgruppe bedeutet,
welche Verbindungen der Formeln (I/a), (I/b) (I/c) und (I/d):

- man gegebenenfalls mit Hilfe einer klassischen Reinigungsmethode reinigt,
- man gewünschtenfalls mit Hilfe einer klassischen Trennmethode in die Enantiomeren auftrennt,
- und die man gegebenenfalls in ihre Additionssalze mit einer pharmazeutisch annehmbaren Säure überführt,
- wobei es sich versteht, daß die Substituenten $R_2$, $R_3$ und $R_4$ im Verlaufe der Synthese der Verbindungen der Formel (I) mit Hilfe klassischer Methoden der organischen Chemie eingeführt oder modifiziert werden können.

6. Verfahren zur Herstellung der Verbindungen der Formel (I) nach Anspruch 1, worin n den Wert 2 besitzt, dadurch gekennzeichnet, daß man als Ausgangsprodukt die Verbindung der Formel (IX) verwendet:

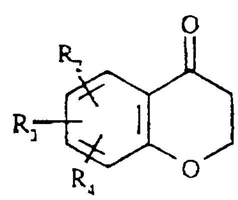

(IX)

in der $R_2$, $R_3$ und $R_4$ die bezüglich der Formel (I) angegebenen Bedeutungen besitzen, welche man mit Methylmagnesiumbromid und dann mit p-Toluolsulfonsäure umsetzt
zur Bildung der Verbindung der Formel (X):

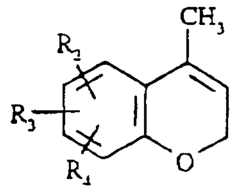

(X)

in der $R_2$, $R_3$ und $R_4$ die bezüglich der Formel (I) angegebenen Bedeutungen besitzen, welche man mit Benzylamin in Gegenwart von Formaldehyd umsetzt, zur Bildung der Verbindung der Formel (XI):

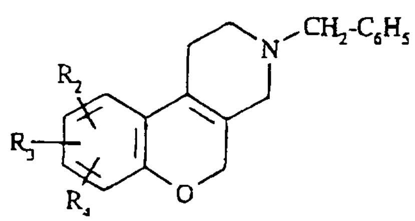

(XI)

in der $R_2$, $R_3$ und $R_4$ die bezüglich der Formel (I) angegebenen Bedeutungen besitzen,
welche anschließend einer katalytischen Hydrierung unterzogen wird zur Bildung der Verbindung der Formel (I/e), einem Sonderfall der Verbindungen der Formel (I):

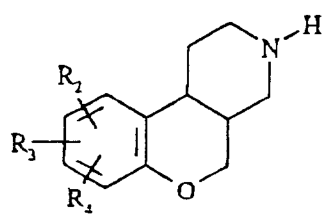

(I/e)

in der $R_2$, $R_3$ und $R_4$ die bezuglich der Formel (I) angegebenen Bedeutungen besitzen,
welche Verbindung der Formel (I/e) man gewünschtenfalls mit einem Halogenderivat der Formel (XII):

$$R'_{1a}\text{-}X \qquad \text{(XII)}$$

in der:

X ein Halogenatom und
$R'_{1a}$ eine geradkettige oder verzweigte ($C_1$-$C_6$)-Alkylgruppe, Acetylgruppe, Benzoylgruppe, Benzylgruppe, Pyridincarbonylgruppe, Pyridinmethylgruppe, 3-Pyridinaminocarbonylgruppe, Phthalimido-($C_1$-$C_6$)-alkylgruppe mit geradkettiger oder verzweigter Alkylgruppe bedeuten,

umsetzt zur Bildung

- entweder der Verbindung der Formel (I/f), einem Sonderfall der Verbindungen der Formel (I):

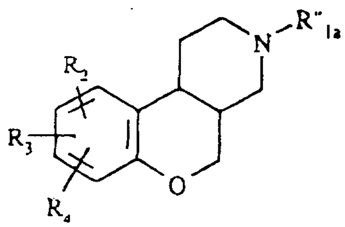

(I/f)

in der $R_2$, $R_3$ und $R_4$ die bezüglich der Formel (I) angegebenen Bedeutungen besitzen und $R''_{1a}$ eine geradkettige oder verzweigte ($C_1$-$C_6$)-Alkylgruppe, Acetylgruppe, Benzoylgruppe, Benzylgruppe, Pyridincarbonylgruppe, Pyridinmethylgruppe, 3-Pyridinaminocarbonylgruppe oder Phthalimido-($C_1$-$C_6$)-alkylgruppe mit geradkettiger oder verzweigter Alkylgruppe bedeutet,

- oder der Verbindung der Formel (XIII):

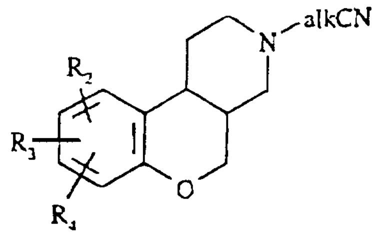

(XIII)

in der $R_2$, $R_3$ und $R_4$ die bezüglich der Formel (I) angegebenen Bedeutungen besitzen und alkCN eine Cyan-($C_1$-$C_6$)-alkylgruppe mit geradkettiger oder verzweigter Alkylgruppe bedeutet,
von welcher Verbindung der Formel (XIII) man die Cyanogruppe zu der Aminogruppe reduziert und welche man mit einem (gegebenenfalls durch ein Halogenatom substituierten) Benzoylhalogenid umsetzt
zur Bildung der Verbindung der Formel (I/g), einem Sonderfall der Verbindungen der Formel (I):

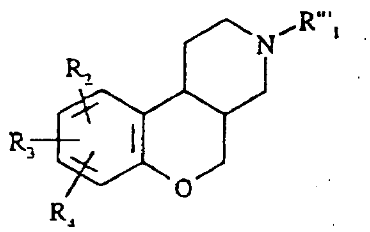

(I/g)

in der $R_2$, $R_3$ und $R_4$ die bezüglich der Formel (I) angegebenen Bedeutungen besitzen und $R'''_1$ eine (gegebenenfalls am Phenylkern durch ein Halogenatom substituierte) Benzoylamino-($C_1$-$C_6$)-alkylgruppe mit geradkettiger oder verzweigter Alkylgruppe darstellt,
welche Verbindungen der Formel (I/e), (I/f) und (I/g):

- man gegebenenfalls mit Hilfe einer klassischen Reinigungsmethode reinigt,
- man gewünschtenfalls mit Hilfe einer klassischen Trennmethode in die Enantiomeren auftrennt,
- und die man gegebenenfalls in ihre Additionssalze mit einer pharmazeutisch annehmbare Säure überführt,
- wobei es sich verstellt, daß die Substituenten $R_2$, $R_3$ und $R_4$ im Verlaufe der Synthese der Verbindungen der Formel (I) mit Hilfe klassischer Methoden der organischen Chemie eingeführt oder modifiziert werden können.

**7.** Pharmazeutische Zubereitungen enthaltend als Wirkstoff mindestens eine Verbindung nach einem der Ansprüche 1 bis 4 allein oder in Kombination mit einem oder mehreren inerten, nichttoxischen, pharmazeutisch annehmbaren Trägermaterialien.

8. Pharmazeutische Zubereitungen nach Anspruch 7 zur Verwendung bei der Behandlung von Krankhelten, die einen Liganden für die Rezeptoren 5-HT2C benötigen, wie der Angst, der Depression, von Störungen der Impulsivität, der Schizophrenie, Appetitstörungen, kardiovaskulären Erkrankungen, Sexualstörungen, zerebraler Ischämie, Drogenmißbrauch, Schlafstörungen und Migräne.